# EUROPEAN PATENT APPLICATION

(11) **EP 2 224 008 A2**
(43) Date of publication of application: **01.09.2010**
(21) Application number: 10166763.2
(22) Date of filing: 10.05.2006
(51) Int. Cl.: C12N 15/31, C12N 15/85, C07K 14/20, C07K 16/12, C12Q 1/68, G01N 33/53, A61K 39/02

(54) **Genes and proteins of brachyspira hyodysenteriae and use of same for diagnosis and therapy**

(30) Priority: 12.05.2005 AU 2005902418
(62) Divisional of application: 06753549.2
(71) Applicant: Murdoch University, Murdoch, Western Australia 6150 (AU); Hampson, David John, Mt. Nasura WA 6112 (AU); Bellgard, Matthew, Attadale 6156 (AU); La, Tom, Beckenham WA 6107 (AU)
(72) Inventor: Hampson, David J, Mt Nasura, Western Australia 6112 (AU); Bellgard, Matthew, Attadale, Western Australia 6156 (AU); LA, Tom, Beckenham,, Western Australia 6107 (AU)
(74) Representative: Dean, John Paul

(57) **Abstract**

Novel polynucleotide and amino acids of *Brachyspira hyodysenteriae* are described. These sequences are useful for diagnosis of *B. hyodysenteriae* disease in animals and as a therapeutic treatment or prophylactic treatment of *B. hyodysenteriae* disease in animals. These sequences may also be useful for diagnostic and therapeutic and/or prophylactic treatment of diseases in animals caused by other *Brachyspira* species, including *B. intermedia, B. alvinipulli, B. aalborgi, B. innocens, B. murdochii, and B. pilosicoli.*

## Description

### Field of Invention

This invention relates to novel genes in *Brachyspira hyodysenteriae* and the proteins encoded therein. This invention further relates to use of these novel genes and proteins for diagnosis of *B. hyodysenteriae* disease, vaccines against *B. hyodysenteriae* and for screening for compounds that kill *B. hyodysenteriae* or block the pathogenic effects of *B. hyodysenteriae.* These sequences may also be useful for diagnostic and therapeutic and/or prophylactic treatment of diseases in animals caused by other *Brachyspira* species, including *B. intermedia, B. alvinipulli, B. aalborgi, B. innocens, B. murdochii, and B. pilosicoli.*

### Background of Invention

Swine dysentery is a significant endemic disease of pigs in Australia and worldwide. Swine dysentery is a contagious mucohaemorrhagic diarrhoeal disease, characterised by extensive inflammation and necrosis of the epithelial surface of the large intestine. Economic losses due to swine dysentery result mainly from growth retardation, costs of medication and mortality. The causative agent of swine dysentery was first identified as an anaerobic spirochaete (then called *Treponema hyodysenteriae*) in 1971, and was recently reassigned to the genus *Brachyspira* as *B. hyodysenteriae.* The disease spectrum for swine dysentery can vary from being mild, transient or unapparent, to being severe and even fatal. Medication strategies in individual piggeries may mask clinical signs and in some piggeries the disease may go unnoticed, or may only be suspected. Whether or not obvious disease occurs, *B. hyodysenteriae* may persist in infected pigs, or in other reservoir hosts such as rodents, or in the environment. All these sources pose potential for transmission of the disease to uninfected herds.

Colonisation by *B. hyodysenteriae* elicits a strong immunological response against the spirochaete, hence indirect evidence of exposure to the spirochaete can be obtained by measuring circulating antibody titres in the blood of infected animals. These antibody titres have been reported to be maintained at low levels, even in animals that have recovered from swine dysentery. Serological tests for detection of antibodies therefore have considerable potential for detecting subclinical infections and recovered carrier pigs that have undetectable numbers of spirochaetes in their large intestines. These tests would be particularly valuable in an easy to use kit form, such as an enzyme-linked immunosorbent assay. A variety of techniques have been developed to demonstrate the presence of circulating antibodies against *B. hyodysenteriae,* including indirect fluorescent antibody tests, haemagglutination tests, microtitration agglutination tests, complement fixation tests, and ELISA using either lipopolysaccharide or whole sonicated spirochaetes as antigen. All these tests have suffered from problems of specificity, as related non-pathogenic intestinal spirochaetes can induce cross-reactive antibodies. These tests are useful for detecting herds where there is obvious disease and high circulating antibody titres, but they are problematic for identifying sub-clinically infected herds and individual infected pigs. Consequently, to date, no completely sensitive and specific assays are available for the detection of antibodies against *B. hyodysenteriae.* The lack of suitable diagnostic tests has hampered control of swine dysentery.

A number of methods are employed to control swine dysentery, varying from the prophylactic use of antimicrobial agents, to complete de-stocking of infected herds and prevention of re-entry of infected carrier pigs. All these options are expensive and, if they are to be fully effective, they require the use of sophisticated diagnostic tests to monitor progress. Currently, detection of swine dysentery herds with sub-clinical infections, and individual healthy carrier animals, remains a major problem and is hampering implementation of effective control measures. A definitive diagnosis of swine dysentery traditionally has required the isolation and identification of *B. hyodysenteriae* from the faeces or mucosa of diseased pigs. Major problems involved include the slow growth and fastidious nutritional requirements of these anaerobic bacteria and confusion due to the presence of morphologically similar spirochaetes in the normal flora of the pig intestine. A significant improvement in the diagnosis of individual affected pigs was achieved with the development of polymerase chain reaction (PCR) assays for the detection of spirochaetes from faeces. Unfortunately in practical applications the limit of detection of PCRs render them unable to detect carrier animals with subclinical infections. As a consequence of these diagnostic problems, there is a clear need to develop a simple and effective diagnostic tool capable of detecting *B. hyodysenteriae* infection at the herd and individual pig level.

A strong immunological response is induced against the spirochaete following colonization with *B. hyodysenteriae,* and pigs recovered from swine dysentery are protected from re-infection. Despite this, attempts to develop vaccines to control swine dysentery have met with very limited success, either because they have provided inadequate protection on a herd basis, or they have been too costly and difficult to produce to make them commercially viable. Bacterin vaccines provide some level of protection, but they tend to be lipopolysaccharide serogroup-specific, which then requires the use of multivalent bacterins. Furthermore they are difficult and costly to produce on a large scale because of the fastidious anaerobic growth requirements of the spirochaete.

Several attempts have been made to develop attenuated live vaccines for swine dysentery. This approach has the disadvantage that attenuated strains show reduced colonisation, and hence cause reduced immune stimulation. Producers and veterinarians are reluctant to use live vaccines for swine dysentery because of the possibility of reversion to virulence, especially as very little is known about genetic regulation and organization in *B. hyodysenteriae*.

The use of recombinant subunit vaccines is an attractive alternative, because the products would be well-defined (essential for registration purposes), and relatively easy to produce on a large scale. To date the first reported use of a recombinant protein from *B. hyodysenteriae* as a vaccine candidate (a 38-kilodalton flagellar protein) failed to prevent colonisation in pigs. This failure is likely to relate specifically to the particular recombinant protein used, as well as to other more down-stream issues of delivery systems and routes, dose rates, choice of adjuvants etc (Gabe, JD, Chang, RJ, Slomiany, R, Andrews, WH and McCaman, MT (1995) Isolation of extracytoplasmic proteins from Serpulina hyodysenteriae B204 and molecular cloning of the flaB1 gene encoding a 38-kilodalton flagellar protein. Infection and Immunity 63:142-148). A more recent reported use of recombinant *B. hyodysenteriae* protein for vaccination was a 30kDa outer membrane lipoprotein (BmpB, also refered to as BlpA) which had homology with the methionine-binding lipoproteins of various pathogenic bacteria. The use of the his-tagged recombinant BmpB for vaccination of pigs, followed by experimental challenge with *B. hyodysenteriae*, resulted in 17-40% of vaccinated pigs developing disease compared to 50-70% of the unvaccinated control pigs developing disease. Because the incidence of disease for the BmpB vaccinated pigs was significantly (P=0.047) less than the control pigs, BmpB appeared to have potential as a swine dysentery vaccine component (La, T, Phillips, ND, Reichel, MP and Hampson, DJ (2004) Protection of pigs from swine dysentery by vaccination with recombinant BmpB, a 29.7 kDa outer-membrane lipoprotein of Brachyspira hyodysenteriae. Veterinary Microbiology 102:97-109). A number of other attempts have been made to identify outer envelop proteins from *B. hyodysenteriae* that could be used as recombinant vaccine components, but again no successful vaccine has yet been made. A much more global approach is needed to the identification of potentially useful immunogenic recombinant proteins from *B. hyodysenteriae.*

To date, only one study using DNA for vaccination has been reported. In this study, the *B. hyodysenteriae ftnA* gene, encoding a putative ferritin, was cloned into an *E. coli* plasmid and the plasmid DNA used to coat gold beads for ballistic vaccination. A murine model for swine dysentery was used to determine the protective nature of vaccination with DNA and/or recombinant protein. Vaccination with recombinant protein induced a good systemic response against ferritin, however vaccination with DNA induced a detectable systemic response only after the final vaccination. Vaccination with DNA followed by a boost with recombinant protein induced a systemic immune response to ferritin only after boosting with protein. However, none of the vaccination regimes tested was able to provide the mice with protection against *B. hyodysenteriae* colonisation and the associated lesions. Interestingly, vaccination of the mice with DNA alone resulted in significant exacerbation of disease (Davis, A.J., Smith, S.C. and Moore, R.J. (2005). The Brachyspira hyodysenteriae ftnA gene: DNA vaccination and real-time PCR quantification of bacteria in a mouse model of disease. Current Microbiology 50: 285-291).

The present invention provides novel *B. hyodysenteriae* amino acid sequences and the polynucleotide sequences that encode them, which have not been identified previously. The present invention also demonstrates that these novel amino acid sequences and DNA sequences can be used for diagnostic assays and vaccines for *B. hyodysenteriae*. These sequences may also be useful for diagnostic and therapeutic and/or prophylactic treatment of diseases in animals caused by other *Brachyspira* species, including *B. intermedia, B. alvinipulli, B. aalborgi, B. innocens, B. murdochii, and B. pilosicoli.*

### Brief Summary of Invention

It is an object of this invention to have novel genes from *B. hyodysenteriae* and the proteins encoded by those genes. It is a further object of this invention that the novel genes and the proteins encoded by those genes can be used for therapeutic and diagnostic purposes. One can use the genes and/or the proteins in a vaccine against *B. hyodysenteriae* and to diagnose *B. hyodysenteriae* infections.

It is an object of this invention to have novel *B. hyodysenteriae* genes having the nucleotide sequence contained in SEQ ID NOs: 1, 3, 5, 7, 9, 11, and 13. It is also an object of this invention to have nucleotide sequences that are homologous to SEQ ID NOs: 1, 3, 5, 7, 9, 11, and 13 where the homology can be 95%, 90%, 85%, 80%, 75% and 70%. This invention also includes a DNA vaccine or DNA immunogenic composition containing the nucleotide sequence of SEQ ID NOs: 1, 3, 5, 7, 9, 11, and 13 and sequences that are 95%, 90%, 85%, 80%, 75% and 70% homologous to these sequences. This invention further includes a diagnostic assay containing DNA having the nucleotide sequence of SEQ ID NOs: 1, 3, 5, 7, 9, 11, and 13 and sequences that are 95%, 90%, 85%, 80%, 75% and 70% homologous to these sequences.

It is also an object of this invention to have plasmids containing DNA having the sequence of SEQ ID NOs: 1, 3, 5, 7, 9, 11, and 13; prokaryotic and/or eukaryotic expression vectors containing DNA having the sequence of SEQ ID NOs: 1, 3, 5, 7, 9, 11, and 13; and a cell containing the plasmids which contain DNA having the sequence of SEQ ID NOs: 1, 3, 5, 7, 9, 11, and 13.

It is an object of this invention to have novel *B. hyodysenteriae* proteins having the amino acid sequence contained in SEQ ID NOs: 2, 4; 6, 8, 10, 12, and 14. It is another object of this invention to have proteins that are 95%, 90%, 85%, 80%, 75% and 70% homologous to the sequences contained in SEQ ID NOs: 2, 4, 6, 8, 10, 12, and 14. It is also an object of this invention for a vaccine or immunogenic composition to contain the proteins having the amino acid sequence contained in SEQ ID NOs: 2, 4, 6, 8, 10, 12, and 14, or amino acid sequences that are 95%, 90%, 85%, 80%, 75% and 70% homologous to the sequences contained in SEQ ID NOs: 2, 4, 6, 8, 10, 12, and 14. It is a further aspect of this invention to have a diagnostic kit containing one or more proteins having a sequence contained in SEQ ID NOs: 2, 4, 6, 8, 10, 12, and 14 or that are 95%, 90%, 85%, 80%, 75% and 70% homologous to the sequences contained in SEQ ID NOs: 2, 4, 6, 8, 10, 12, and 14.

It is another aspect of this invention to have nucleotide sequences which encode the proteins having the amino acid sequence contained in SEQ ID NOs: 2, 4, 6, 8, 10, 12, and 14. The invention also covers plasmids, eukaryotic and prokaryotic expression vectors, and DNA vaccines which contain DNA having a sequence which encodes a protein having the amino acid sequence contained in SEQ ID NOs: 2, 4, 6, 8, 10, 12, and 14. Cells which contain these plasmids and expression vectors are included in this invention.

This invention includes monoclonal antibodies that bind to proteins having an amino acid sequence contained in SEQ ID NOs: 2, 4, 6, 8, 10, 12, and 14 or bind to proteins that are 95%, 90%, 85%, 80%, 75% and 70% homologous to the sequences contained in SEQ ID NOs: 2, 4, 6, 8, 10, 12, and 14. Diagnostic kits containing the monoclonal antibodies that bind to proteins having an amino acid sequence contained in SEQ ID NOs: 2, 4, 6, 8, 10, 12, and 14 or bind to proteins that are 95%, 90%, 85%, 80%, 75% and 70% homologous to the sequences contained in SEQ ID NOs: 2, 4, 6, 8, 10, 12, and 14 are included in this invention. These diagnostic kits can detect the presence of *B. hyodysenteriae* in an animal. The animal is preferably any mammal and bird; more preferably, chicken, goose, duck, turkey, parakeet, dog, cat, hamster, gerbil, rabbit, ferret, horse, cow, sheep, pig, monkey, and human.

The invention also contemplates the method of preventing or treating an infection of *B. hyodysenteriae* in an animal by administering to an animal a DNA vaccine containing one or more nucleotide sequences listed in SEQ ID NOs: 1, 3, 5, 7, 9, 11, and 13 or sequences that are 95%, 90%, 85%, 80%, 75% and 70% homologous to these sequences. This invention also covers a method of preventing or treating an infection *of B. hyodysenteriae* in an animal by administering to an animal a vaccine containing one or more proteins having the amino acid sequence containing in SEQ ID NOs: 2, 4, 6, 8, 10, 12, and 14 or sequences that are 95%, 90%, 85%, 80%, 75% and 70% homologous to these sequences. The animal is preferably any mammal and bird; more preferably, chicken, goose, duck, turkey, parakeet, dog, cat, hamster, gerbil, rabbit, ferret, horse, cow, sheep, pig, monkey, and human.

The invention also contemplates the method of generating an immune response in an animal by administering to an animal an immunogenic composition containing one or more nucleotide sequences listed in SEQ ID NOs: 1, 3, 5, 7, 9, 11, and 13 or sequences that are 95%, 90%, 85%, 80%, 75% and 70% homologous to these sequences. This invention also covers a method of generating an immune response in an animal by administering to an animal an immunogenic composition containing one or more proteins having the amino acid sequence containing in SEQ ID NOs: 2, 4, 6, 8, 10, 12, and 14 or sequences that are 95%, 90%, 85%, 80%, 75% and 70% homologous to these sequences. The animal is preferably any mammal and bird; more preferably, chicken, goose, duck, turkey, parakeet, dog, cat, hamster, gerbil, rabbit, ferret, horse, cow, sheep, pig, monkey, and human.

### Detailed Summary of Invention

The articles "a" and "an" are used herein to refer to one or to more than one (i.e., to at least one) of the grammatical object of the article. By way of example, "an element" means one element or more than one element.

The term "amino acid" is intended to embrace all molecules, whether natural or synthetic, which include both an amino functionality and an acid functionality and capable of being included in a polymer of naturally-occurring amino acids. Exemplary amino acids include naturally-occurring amino acids; analogs, derivatives and congeners thereof; amino acid analogs having variant side chains; and all stereoisomers of any of any of the foregoing.

An animal can be any mammal or bird. Examples of mammals include dog, cat, hamster, gerbil, rabbit, ferret, horse, cow, sheep, pig, monkey, and human. Examples of birds include chicken, goose, duck, turkey, and parakeet.

The term "conserved residue" refers to an amino acid that is a member of a group of amino acids having certain common properties. The term "conservative amino acid substitution" refers to the substitution (conceptually or otherwise) of an amino acid from one such group with a different amino acid from the same group. A functional way to define common properties between individual amino acids is to analyze the normalized frequencies of amino acid changes between corresponding proteins of homologous organisms (Schulz, G. E. and R. H. Schinner., Principles of Protein Structure, Springer-Verlag). According to such analyses, groups of amino acids may be defined where amino acids within a group exchange preferentially with each other, and therefore resemble each other most in their impact on the overall protein structure (Schulz, G. E. and R. H. Schirmer, Principles of Protein Structure, Springer-Verlag). Examples of amino acid groups defined in this manner include: (i) a positively-charged group containing Lys, Arg and His, (ii) a negatively-charged group containing Glu and Asp, (iii) an aromatic group containing Phe, Tyr and Trp, (iv) a nitrogen ring group containing His and Trp, (v) a large aliphatic nonpolar group containing Val, Leu and De, (vi) a slightly-polar group containing Met and Cys, (vii) a small-residue group containing Ser, Thr, Asp, Asn, Gly, Ala, Glu, Gln and Pro, (viii) an aliphatic group containing Val, Leu, De, Met and Cys, and (ix) a small, hydroxyl group containing Ser and Thr.

A "fusion protein" or "fusion polypeptide" refers to a chimeric protein as that term is known in the art and may be constructed using methods known in the art. In many examples of fusion proteins, there are two different polypeptide sequences, and in certain cases, there may be more. The polynucleotide sequences encoding the fusion protein may be operably linked in frame so that the fusion protein may be translated correctly. A fusion protein may include polypeptide sequences from the same species or from different species. In various embodiments, the fusion polypeptide may contain one or more amino acid sequences linked to a first polypeptide. In the case where more than one amino acid sequence is fused to a first polypeptide, the fusion sequences may be multiple copies of the same sequence, or alternatively, may be different amino acid sequences. The fusion polypeptides may be fused to the N-terminus, the C-terminus, or the N- and C-terminus of the first polypeptide. Exemplary fusion proteins include polypeptides containing a glutathione S-transferase tag (GST-tag), histidine tag (His-tag), an immunoglobulin domain or an immunoglobulin binding domain.

The term "isolated polypeptide" refers to a polypeptide, in certain embodiments prepared from recombinant DNA or RNA, or of synthetic origin or natural origin, or some combination thereof, which (1) is not associated with proteins that it is normally found with in nature, (2) is separated from the cell in which it normally occurs, (3) is free of other proteins from the same cellular source, (4) is expressed by a cell from a different species, or (5) does not occur in nature. It is possible for an isolated polypeptide to exist but not qualify as a purified polypeptide.

The term "isolated nucleic acid" and "isolated polynucleotide" refers to a polynucleotide whether genomic DNA, cDNA, mRNA, tRNA, rRNA, iRNA, or a polynucleotide obtained from a cellular organelie (such as mitochondria and chloroplast), or whether from synthetic origin, which (1) is not associated with the cell in which the "isolated nucleic acid" is found in nature, or (2) is operably linked to a polynucleotide to which it is not linked in nature. It is possible for an isolated polynucleotide to exist but not qualify as a purified polynucleotide.

The term "nucleic acid" and "polynucleotide" refers to a polymeric form of nucleotides, either ribonucleotides or deoxyribonucleotides or a modified form of either type of nucleotide. The terms should also be understood to include, as equivalents, analogs of either RNA or DNA made from nucleotide analogs, and, as applicable to the embodiment being described, single-stranded (such as sense or antisense) and double-stranded polynucleotides.

The term "nucleic acid of the invention" and "polynucleotide of the invention" refers to a nucleic acid encoding a polypeptide of the invention. A polynucleotide of the invention may comprise all, or a portion of, a subject nucleic acid sequence; a nucleotide sequence at least 60%, 70%, 80%, 90%, 95%, 96%, 97%, 98% or 99% identical to a subject nucleic acid sequence; a nucleotide sequence that hybridizes under stringent conditions to a subject nucleic acid sequence; nucleotide sequences encoding polypeptides that are functionally equivalent to polypeptides of the invention; nucleotide sequences encoding polypeptides at least about 60%, 70%, 80%, 85%, 90%, 95%, 98%, 99% homologous or identical with a subject amino acid sequence; nucleotide sequences encoding polypeptides having an activity of a polypeptide of the invention and having at least about 60%, 70%, 80%, 85%, 90%, 95%, 98%, 99% or more homology or identity with a subject amino acid sequence; nucleotide sequences that differ by 1 to about 2, 3, 5, 7, 10, 15, 20, 30, 50, 75 or more nucleotide substitutions, additions or deletions, such as allelic variants, of a subject nucleic acid sequence; nucleic acids derived from and evolutionarily related to a subject nucleic acid sequence; and complements of, and nucleotide sequences resulting from the degeneracy of the genetic code, for all of the foregoing and other nucleic acids of the invention. Nucleic acids of the invention also include homologs, *e*.*g*., orthologs and paralogs, of a subject nucleic acid sequence and also variants of a subject nucleic acid sequence which have been codon optimized for expression in a particular organism (*e*.*g*., host cell).

The term "operably linked", when describing the relationship between two nucleic acid regions, refers to a juxtaposition wherein the regions are in a relationship permitting them to function in their intended manner. For example, a control sequence "operably linked" to a coding sequence is ligated in such a way that expression of the coding sequence is achieved under conditions compatible with the control sequences, such as when the appropriate molecules (e.g., inducers and polymerases) are bound to the control or regulatory sequence(s).

The term "polypeptide", and the terms "protein" and "peptide" which are used interchangeably herein, refers to a polymer of amino acids. Exemplary polypeptides include gene products, naturally-occurring proteins, homologs, orthologs, paralogs, fragments, and other equivalents, variants and analogs of the foregoing.

The terms "polypeptide fragment" or "fragment", when used in reference to a reference polypeptide, refers to a polypeptide in which amino acid residues are deleted as compared to the reference polypeptide itself, but where the remaining amino acid sequence is usually identical to the corresponding positions in the reference polypeptide. Such deletions may occur at the amino-terminus or carboxy-terminus of the reference polypeptide, or alternatively both. Fragments typically are at least 5, 6, 8 or 10 amino acids long, at least 14 amino acids long, at least 20, 30, 40 or 50 amino acids long, at least 75 amino acids long, or at least 100, 150,200,300,500 or more amino acids long. A fragment can retain one or more of the biological activities of the reference polypeptide. In certain embodiments, a fragment may comprise a domain having the desired biological activity, and optionally additional amino acids on one or both sides of the domain, which additional amino acids may number from 5, 10, 15, 20, 30, 40, 50, or up to 100 or more residues. Further, fragments can include a sub-fragment of a specific region, which sub-fragment retains a function of the region from which it is derived. In another embodiment, a fragment may have immunogenic properties.

The term "polypeptide of the invention" refers to a polypeptide containing a subject amino acid sequence, or an equivalent or fragment thereof. Polypeptides of the invention include polypeptides containing all or a portion of a subject amino acid sequence; a subject amino acid sequence with 1 to about 2, 3, 5, 7, 10, 15, 20, 30, 50, 75 or more conservative amino acid substitutions; an amino acid sequence that is at least 60%, 70%, 80%, 90%, 95%, 96%, 97%, 98%, or 99% identical to a subject amino acid sequence; and functional fragments thereof. Polypeptides of the invention also include homologs, *e*.*g*., orthologs and paralogs, of a subject amino acid sequence.

It is also possible to modify the structure of the polypeptides of the invention for such purposes as enhancing therapeutic or prophylactic efficacy, or stability (e.g., *ex vivo* shelf life, resistance to proteolytic degradation *in vivo, etc*.). Such modified polypeptides, when designed to retain at least one activity of the naturally-occurring form of the protein, are considered "functional equivalents" of the polypeptides described in more detail herein. Such modified polypeptides may be produced, for instance, by amino acid substitution, deletion, or addition, which substitutions may consist in whole or part by conservative amino acid substitutions.

For instance, it is reasonable to expect that an isolated conservative amino acid substitution, such as replacement of a leucine with an isoleucine or valine, an aspartate with a glutamate, a threonine with a serine, will not have a major affect on the biological activity of the resulting molecule. Whether a change in the amino acid sequence of a polypeptide results in a functional homolog may be readily determined by assessing the ability of the variant polypeptide to produce a response similar to that of the wild-type protein. Polypeptides in which more than one replacement has taken place may readily be tested in the same manner.

The term "purified" refers to an object species that is the predominant species present (*i*.*e*., on a molar basis it is more abundant than any other individual species in the composition). A "purified fraction" is a composition wherein the object species is at least about 50 percent (on a molar basis) of all species present. In making the determination of the purity or a species in solution or dispersion, the solvent or matrix in which the species is dissolved or dispersed is usually not included in such determination; instead, only the species (including the one of interest) dissolved or dispersed are taken into account. Generally, a purified composition will have one species that is more than about 80% of all species present in the composition, more than about 85%, 90%, 95%, 99% or more of all species present. The object species may be purified to essential homogeneity (contaminant species cannot be detected in the composition by conventional detection methods) wherein the composition is essentially a single species. A skilled artisan may purify a polypeptide of the invention using standard techniques for protein purification in light of the teachings herein. Purity of a polypeptide may be determined by a number of methods known to those of skill in the art, including for example, amino-terminal amino acid sequence analysis, gel electrophoresis, mass-spectrometry analysis and the methods described herein.

The terms "recombinant protein" or "recombinant polypeptide" refer to a polypeptide which is produced by recombinant DNA techniques. An example of such techniques includes the case when DNA encoding the expressed protein is inserted into a suitable expression vector which is in turn used to transform a host cell to produce the protein or polypeptide encoded by the DNA.

The term "regulatory sequence" is a generic term used throughout the specification to refer to polynucleotide sequences, such as initiation signals, enhancers, regulators and promoters, that are necessary or desirable to affect the expression of coding and non-coding sequences to which they are operably linked. Exemplary regulatory sequences are described in Goeddel; Gene Expression Technology: Methods in Enzymology, Academic Press, San Diego, CA (1990), and include, for example, the early and late promoters of SV40, adenovirus or cytomegalovirus immediate early promoter, the lac system, the trp system, the TAC or TRC system, T7 promoter whose expression is directed by T7 RNA polymerase, the major operator and promoter regions of phage lambda, the control regions for fd coat protein, the promoter for 3-phosphoglycerate kinase or other glycolytic enzymes, the promoters of acid phosphatase (*e*.*g*., Pho5), the promoters of the yeast α-mating factors, the polyhedron promoter of the baculovirus system and other sequences known to control the expression of genes of prokaryotic or eukaryotic cells or their viruses, and various combinations thereof. The nature and use of such control sequences may differ depending upon the host organism. In prokaryotes, such regulatory sequences generally include promoter, ribosomal binding site, and transcription termination sequences. The term "regulatory sequence" is intended to include, at a minimum, components whose presence may influence expression, and may also include additional components whose presence is advantageous, for example, leader sequences and fusion partner sequences. In certain embodiments, transcription of a polynucleotide sequence is under the control of a promoter sequence (or other regulatory sequence) which controls the expression of the polynucleotide in a cell-type in which expression is intended. It will also be understood that the polynucleotide can be under the control of regulatory sequences which are the same or different from those sequences which control expression of the naturally-occurring form of the polynucleotide.

The term "sequence homology" refers to the proportion of base matches between two nucleic acid sequences or the proportion of amino acid matches between two amino acid sequences. When sequence homology is expressed as a percentage, *e*.*g*., 50%, the percentage denotes the proportion of matches over the length of sequence from a desired sequence that is compared to some other sequence. Gaps (in either of the two sequences) are permitted to maximize matching; gap lengths of 15 bases or less are usually used, 6 bases or less are used more frequently, with 2 bases or less used even more frequently. The term "sequence identity" means that sequences are identical (*i*.*e*., on a nucleotide-by-nucleotide basis for nucleic acids or amino acid-by-amino acid basis for polypeptides) over a window of comparison. The term "percentage of sequence identity" is calculated by comparing two optimally aligned sequences over the comparison window, determining the number of positions at which the identical amino acids or nucleotides occurs in both sequences to yield the number of matched positions, dividing the number of matched positions by the total number of positions in the comparison window, and multiplying the result by 100 to yield the percentage of sequence identity. Methods to calculate sequence identity are known to those of skill in the art and described in further detail below.

The term "soluble" as used herein with reference to a polypeptide of the invention or other protein, means that upon expression in cell culture, at least some portion of the polypeptide or protein expressed remains in the cytoplasmic fraction of the cell and does not fractionate with the cellular debris upon lysis and centrifugation of the lysate. Solubility of a polypeptide may be increased by a variety of art recognized methods, including fusion to a heterologous amino acid sequence, deletion of amino acid residues, amino acid substitution (*e*.*g*., enriching the sequence with amino acid residues having hydrophilic side chains), and chemical modification (e.g., addition of hydrophilic groups).

The solubility of polypeptides may be measured using a variety of art recognized techniques, including, dynamic light scattering to determine aggregation state, UV absorption, centrifugation to separate aggregated from non-aggregated material, and SDS gel electrophoresis (*e*.*g*., the amount of protein in the soluble fraction is compared to the amount of protein in the soluble and insoluble fractions combined). When expressed in a host cell, the polypeptides of the invention may be at least about 1%, 2%, 5%, 10%, 20%, 30%, 40%, 50%, 60%, 70%, 80%, 90% or more soluble, e.g., at least about 1%, 2%, 5%, 10%, 20%, 30%, 40%, 50%, 60%, 70%, 80%, 90% or more of the total amount of protein expressed in the cell is found in the cytoplasmic fraction. In certain embodiments, a one liter culture of cells expressing a polypeptide of the invention will produce at least about 0.1, 0.2, 0.5, 1, 2, 5, 10, 20, 30, 40, 50 milligrams of more of soluble protein. In an exemplary embodiment, a polypeptide of the invention is at least about 10% soluble and will produce at least about 1 milligram of protein from a one liter cell culture.

The term "specifically hybridizes" refers to detectable and specific nucleic acid binding. Polynucleotides, oligonucleotides and nucleic acids of the invention selectively hybridize to nucleic acid strands under hybridization and wash conditions that minimize appreciable amounts of detectable binding to nonspecific nucleic acids. Stringent conditions may be used to achieve selective hybridization conditions as known in the art and discussed herein. Generally, the nucleic acid sequence homology between the polynucleotides, oligonucleotides, and nucleic acids of the invention and a nucleic acid sequence of interest will be at least 30%, 40%, 50%, 60%, 70%, 80%, 85%, 90%, 95%, 98%, 99%, or more. In certain instances, hybridization and washing conditions are performed under stringent conditions according to conventional hybridization procedures and as described further herein.

The terms "stringent conditions" or "stringent hybridization conditions" refer to conditions which promote specific hybridization between two complementary polynucleotide strands so as to form a duplex. Stringent conditions may be selected to be about 5°C lower than the thermal melting point (Tm) for a given polynucleotide duplex at a defined ionic strength and pH. The length of the complementary polynucleotide strands and their GC content will determine the Tm of the duplex, and thus the hybridization conditions necessary for obtaining a desired specificity of hybridization. The Tm is the temperature (under defined ionic strength and pH) at which 50% of a polynucleotide sequence hybridizes to a perfectly matched complementary strand. In certain cases it may be desirable to increase the stringency of the hybridization conditions to be about equal to the Tm for a particular duplex.

A variety of techniques for estimating the Tm are available. Typically, G-C base pairs in a duplex are estimated to contribute about 3°C to the Tm, while A-T base pairs are estimated to contribute about 2°C, up to a theoretical maximum of about 80-100°C.

However, more sophisticated models of Tm are available in which G-C stacking interactions, solvent effects, the desired assay temperature and the like are taken into account. For example, probes can be designed to have a dissociation temperature (Td) of approximately 60°C, using the formula: Td = (((3 x #GC) + (2 x #AT)) x 37) - 562)/#bp) - 5; where #GC, #AT, and #bp are the number of guanine-cytosine base pairs, the number of adenine-thymine base pairs, and the number of total base pairs, respectively, involved in the formation of the duplex.

Hybridization may be carried out in 5x SSC, 4x SSC, 3x SSC, 2x SSC, 1x SSC or 0.2x SSC for at least about 1 hour, 2 hours, 5 hours, 12 hours, or 24 hours. The temperature of the hybridization may be increased to adjust the stringency of the reaction, for example, from about 25°C (room temperature), to about 45°C, 50°C, 55°C, 60°C, or 65°C. The hybridization reaction may also include another agent affecting the stringency, for example, hybridization conducted in the presence of 50% formamide increases the stringency of hybridization at a defined temperature.

The hybridization reaction may be followed by a single wash step, or two or more wash steps, which may be at the same or a different salinity and temperature. For example, the temperature of the wash may be increased to adjust the stringency from about 25°C (room temperature), to about 45°C, 50°C, 55°C, 60°C, 65°C, or higher. The wash step may be conducted in the presence of a detergent, *e*.*g*., 0.1 or 0.2% SDS. For example, hybridization may be followed by two wash steps at 65°C each for about 20 minutes in 2x SSC, 0.1% SDS, and optionally two additional wash steps at 65°C each for about 20 minutes in 0.2x SSC, 0.1%SDS.

Exemplary stringent hybridization conditions include overnight hybridization at 65°C in a solution containing 50% formamide, 10x Denhardt (0.2% Ficoll, 0.2% polyvinylpyrrolidone, 0.2% bovine serum albumin) and 200 µg/ml of denatured carrier DNA, *e*.*g*., sheared salmon sperm DNA, followed by two wash steps at 65°C each for about 20 minutes in 2x SSC, 0.1% SDS, and two wash steps at 65°C each for about 20 minutes in 0.2x SSC, 0.1 % SDS.

Hybridization may consist of hybridizing two nucleic acids in solution, or a nucleic acid in solution to a nucleic acid attached to a solid support, *e*.*g*., a filter. When one nucleic acid is on a solid support, a prehybridization step may be conducted prior to hybridization. Prehybridization may be carried out for at least about 1 hour, 3 hours or 10 hours in the same solution and at the same temperature as the hybridization solution (without the complementary polynucleotide strand).

Appropriate stringency conditions are known to those skilled in the art or may be determined experimentally by the skilled artisan. See, for example, Current Protocols in Molecular Biology, John Wiley & Sons, N.Y. (1989), 6.3.1-12.3.6; Sambrook et al., 1989, Molecular Cloning, A Laboratory Manual, Cold Spring Harbor Press, N.Y.; S. Agrawal (ed.) Methods in Molecular Biology, volume 20; Tijssen (1993) Laboratory Techniques in Biochemistry and Molecular Biology -- Hybridization With Nucleic Acid Probes, e.g., part I chapter 2 "Overview of principles of hybridization and the strategy of nucleic acid probe assays", Elsevier, New York; and Tibanyenda, N. et al., Eur. J. Biochem. 139:19 (1984) and Ebel, S. et al., Biochem. 31:12083 (1992).

The term "vector" refers to a nucleic acid capable of transporting another nucleic acid to which it has been linked. One type of vector which may be used in accord with the invention is an episome, i.e., a nucleic acid capable of extra-chromosomal replication. Other vectors include those capable of autonomous replication and expression of nucleic acids to which they are linked. Vectors capable of directing the expression of genes to which they are operatively linked are referred to herein as "expression vectors". In general, expression vectors of utility in recombinant DNA techniques are often in the form of "plasmids" which refer to circular double stranded DNA molecules which, in their vector form are not bound to the chromosome. In the present specification, "plasmid" and "vector" are used interchangeably as the plasmid is the most commonly used form of vector. However, the invention is intended to include such other forms of expression vectors which serve equivalent functions and which become known in the art subsequently hereto.

The nucleic acids of the invention may be used as diagnostic reagents to detect the presence or absence of the target DNA or RNA sequences to which they specifically bind, such as for determining the level of expression of a nucleic acid of the invention. In one aspect, the present invention contemplates a method for detecting the presence of a nucleic acid of the invention or a portion thereof in a sample, the method of the steps of: (a) providing an oligonucleotide at least eight nucleotides in length, the oligonucleotide being complementary to a portion of a nucleic acid of the invention; (b) contacting the oligonucleotide with a sample containing at least one nucleic acid under conditions that permit hybridization of the oligonucleotide with a nucleic acid of the invention or a portion thereof; and (c) detecting hybridization of the oligonucleotide to a nucleic acid in the sample, thereby detecting the presence of a nucleic acid of the invention or a portion thereof in the sample. In another aspect, the present invention contemplates a method for detecting the presence of a nucleic acid of the invention or a portion thereof in a sample, by (a) providing a pair of single stranded oligonucleotides, each of which is at least eight nucleotides in length, complementary to sequences of a nucleic acid of the invention, and wherein the sequences to which the oligonucleotides are complementary are at least ten nucleotides apart; and (b) contacting the oligonucleotides with a sample containing at least one nucleic acid under hybridization conditions; (c) amplifying the nucleotide sequence between the two oligonucleotide primers; and (d) detecting the presence of the amplified sequence, thereby detecting the presence of a nucleic acid of the invention or a portion thereof in the sample.

In another aspect of the invention, the polynucleotide of the invention is provided in an expression vector containing a nucleotide sequence encoding a polypeptide of the invention and operably linked to at least one regulatory sequence. It should be understood that the design of the expression vector may depend on such factors as the choice of the host cell to be transformed and/or the type of protein desired to be expressed. The vector's copy number, the ability to control that copy number and the expression of any other protein encoded by the vector, such as antibiotic markers, should be considered.

An expression vector containing the polynucleotide of the invention can then be used as a pharmaceutical agent to treat an animal infected with *B. hyodysenteriae* or as a vaccine (also a pharmaceutical agent) to prevent an animal from being infected with *B. hyodysenteriae*, or to reduce the symptoms and course of the disease if the animal does become infected. One manner of using an expression vector as a pharmaceutical agent is to administer a nucleic acid vaccine to the animal at risk of being infected or to the animal after being infected. Nucleic acid vaccine technology is well-described in the art. Some descriptions can be found in U.S. Patent 6,562,376 (Hooper et al.); U.S. Patent 5,589,466 (Felgner, et al.); U.S. Patent 6,673,776 (Feigner, et al.); and U.S. Patent 6,710,035 (Felgner, et al.). Nucleic acid vaccines can be injected into muscle or intradermally, can be electroporated into the animal (see WO 01/23537, King et al.; and WO 01/68889, Malone et al.), via lipid compositions (see U.S. Patent 5,703,055, Felgner, et al), or other mechanisms known in the art field.

Expression vectors can also be transfected into bacteria which can be administered to the target animal to induce an immune response to the protein encoded by the nucleotides of this invention contained on the expression vector. The expression vector can contain eukaryotic expression sequences such that the nucleotides of this invention are transcribed and translated in the host animal. Alternatively, the expression vector can be transcribed in the bacteria and then translated in the host animal. The bacteria used as a carrier of the expression vector should be attenuated but still invasive. One can use *Shigella spp., Salmonella spp., Escherichia spp.,* and *Aeromonas spp.,* just to name a few, that have been attenuated but still invasive. Examples of these methods can be found in U.S. Patent 5,824,538 (Branstrom et al); U.S. Patent 5,877,159 (Powell, et al.); U.S. Patent 6,150,170 (Powell, et al.); U.S. Patent 6,500,419 (Hone, et al.); and U.S. Patent 6,682,729 (Powell, et al.).

Alternatively, the polynucleotides of this invention can be placed in certain viruses which act a vector. Viral vectors can either express the proteins of this invention on the surface of the virus, or carry polynucleotides of this invention into an animal cell where the polynucleotide is transcribed and translated into a protein. The animal infected with the viral vectors can develop an immune response to the proteins encoded by the polynucleotides of this invention. Thereby one can alleviate or prevent an infection by *B. hyodysenteriae* in the animal which received the viral vectors. Examples of viral vectors can be found U.S. Patent 5,283,191 (Morgan et al.); U.S. Patent 5,554,525 (Sondermeijer et al) and U.S. Patent 5,712,118 (Murphy).

The polynucleotide of the invention may be used to cause expression and over-expression of a polypeptide of the invention in cells propagated in culture, *e*.*g*. to produce proteins or polypeptides, including fusion proteins or polypeptides.

This invention pertains to a host cell transfected with a recombinant gene in order to express a polypeptide of the invention. The host cell may be any prokaryotic or eukaryotic cell. For example, a polypeptide of the invention may be expressed in bacterial cells, such as *E. coli,* insect cells (baculovirus), yeast, plant, or mammalian cells. In those instances when the host cell is human, it may or may not be in a live subject. Other suitable host cells are known to those skilled in the art. Additionally, the host cell may be supplemented with tRNA molecules not typically found in the host so as to optimize expression of the polypeptide. Alternatively, the nucleotide sequence may be altered to optimize expression in the host cell, yet the protein produced would have high homology to the originally encoded protein. Other methods suitable for maximizing expression of the polypeptide will be known to those in the art.

The present invention further pertains to methods of producing the polypeptides of the invention. For example, a host cell transfected with an expression vector encoding a polypeptide of the invention may be cultured under appropriate conditions to allow expression of the polypeptide to occur. The polypeptide may be secreted and isolated from a mixture of cells and medium containing the polypeptide. Alternatively, the polypeptide may be retained cytoplasmically and the cells harvested, lysed and the protein isolated.

A cell culture includes host cells, media and other byproducts. Suitable media for cell culture are well known in the art. The polypeptide may be isolated from cell culture medium, host cells, or both using techniques known in the art for purifying proteins, including ionexchange chromatography, gel filtration chromatography, ultrafiltration, electrophoresis, and immunoaffinity purification with antibodies specific for particular epitopes of a polypeptide of the invention.

Thus, a nucleotide sequence encoding all or a selected portion of polypeptide of the invention, may be used to produce a recombinant form of the protein via microbial or eukaryotic cellular processes. Ligating the sequence into a polynucleotide construct, such as an expression vector, and transforming or transfecting into hosts, either eukaryotic (yeast, avian, insect or mammalian) or prokaryotic (bacterial cells), are standard procedures. Similar procedures, or modifications thereof, may be employed to prepare recombinant polypeptides of the invention by microbial means or tissue-culture technology.

Suitable vectors for the expression of a polypeptide of the invention include plasmids of the types: pTrcHis-derived plasmids, pET-derived plasmids, pBR322-derived plasmids, pEMBL-derived plasmids, pEX-derived plasmids, pBTac-derived plasmids and pUC-derived plasmids for expression in prokaryotic cells, such as *E. coli.* The various methods employed in the preparation of the plasmids and transformation of host organisms are well known in the art. For other suitable expression systems for both prokaryotic and eukaryotic cells, as well as general recombinant procedures, see Molecular Cloning, A Laboratory Manual, 2nd Ed., ed. by Sambrook, Fritsch and Maniatis (Cold Spring Harbor Laboratory Press, 1989) Chapters 16 and 17.

Coding sequences for a polypeptide of interest may be incorporated as a part of a fusion gene including a nucleotide sequence encoding a different polypeptide. The present invention contemplates an isolated polynucleotide containing a nucleic acid of the invention and at least one heterologous sequence encoding a heterologous peptide linked in frame to the nucleotide sequence of the nucleic acid of the invention so as to encode a fusion protein containing the heterologous polypeptide. The heterologous polypeptide may be fused to (a) the C-terminus of the polypeptide of the invention, (b) the N-terminus of the polypeptide of the invention, or (c) the C-terininus and the N-terminus of the polypeptide of the invention. In certain instances, the heterologous sequence encodes a polypeptide permitting the detection, isolation, solubilization and/or stabilization of the polypeptide to which it is fused. In still other embodiments, the heterologous sequence encodes a polypeptide such as a poly His tag, myc, HA, GST, protein A, protein G, calmodulin-binding peptide, thioredoxin, maltose-binding protein, poly arginine, poly His-Asp, FLAG, a portion of an immunoglobulin protein, and a transcytosis peptide.

Fusion expression systems can be useful when it is desirable to produce an immunogenic fragment of a polypeptide of the invention. For example, the VP6 capsid protein of rotavirus may be used as an immunologic carrier protein for portions of polypeptide, either in the monomeric form or in the form of a viral particle. The nucleic acid sequences corresponding to the portion of a polypeptide of the invention to which antibodies are to be raised may be incorporated into a fusion gene construct which includes coding sequences for a late vaccinia virus structural protein to produce a set of recombinant viruses expressing fusion proteins comprising a portion of the protein as part of the virion. The Hepatitis B surface antigen may also be utilized in this role as well. Similarly, chimeric constructs coding for fusion proteins containing a portion of a polypeptide of the invention and the poliovirus capsid protein may be created to enhance immunogenicity (see, for example, EP Publication NO: 0259149; and Evans et al., (1989) Nature 339:385; Huang et al., (1988) J. Virol. 62:3855; and Schlienger et al., (1992) J. Virol. 66:2).

Fusion proteins may facilitate the expression and/or purification of proteins. For example, a polypeptide of the invention may be generated as a glutathione-S-transferase (GST) fusion protein. Such GST fusion proteins may be used to simplify purification of a polypeptide of the invention, such as through the use of glutathione-derivatized matrices (see, for example, Current Protocols in Molecular Biology, eds. Ausubel et al., (N.Y.: John Wiley & Sons, 1991)). In another embodiment, a fusion gene coding for a purification leader sequence, such as a poly-(His)/enterokinase cleavage site sequence at the N-terminus of the desired portion of the recombinant protein, may allow purification of the expressed fusion protein by affinity chromatography using a Ni²⁺ metal resin. The purification leader sequence may then be subsequently removed by treatment with enterokinase to provide the purified protein (e.g., see Hochuli et al., (1987) J. Chromatography 411: 177; and Janknecht et al., PNAS USA 88:8972).

Techniques for making fusion genes are well known. Essentially, the joining of various DNA fragments coding for different polypeptide sequences is performed in accordance with conventional techniques, employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene may be synthesized by conventional techniques including automated DNA synthesizers. Alternatively, PCR amplification of gene fragments may be carried out using anchor primers which give rise to complementary overhangs between two consecutive gene fragments which may subsequently be annealed to generate a chimeric gene sequence (see, for example, Current Protocols in Molecular Biology, eds. Ausubel et aI., John Wiley & Sons: 1992).

In other embodiments, the invention provides for nucleic acids of the invention immobilized onto a solid surface, including, plates, microtiter plates, slides, beads, particles, spheres, films, strands, precipitates, gels, sheets, tubing, containers, capillaries, pads, slices, *etc.* The nucleic acids of the invention may be immobilized onto a chip as part of an array. The array may contain one or more polynucleotides of the invention as described herein. In one embodiment, the chip contains one or more polynucleotides of the invention as part of an array of polynucleotide sequences from the same pathogenic species as such polynucleotide(s).

In a preferred form of the invention there is provided isolated *B. hyodysenteriae* polypeptides as herein described, and also the polynucleotide sequences encoding these polypeptides. More desirably the *B. hyodysenteriae* polypeptides are provided in substantially purified form.

Preferred polypeptides of the invention will have one or more biological properties (e.g., *in vivo, in vitro* or immunological properties) of the native full-length polypeptide. Non-functional polypeptides are also included within the scope of the invention because they may be useful, for example, as antagonists of the functional polypeptides. The biological properties of analogues, fragments, or derivatives relative to wild type may be determined, for example, by means of biological assays.

Polypeptides, including analogues, fragments and derivatives, can be prepared synthetically (*e*.*g*., using the well known techniques of solid phase or solution phase peptide synthesis). Preferably, solid phase synthetic techniques are employed. Alternatively, the polypeptides of the invention can be prepared using well known genetic engineering techniques, as described *infra.* In yet another embodiment, the polypeptides can be purified (*e*.*g*., by immunoaffinity purification) from a biological fluid, such as but not limited to plasma, faeces, serum, or urine from animals, including, but not limited to, pig, chicken, goose, duck, turkey, parakeet, human, monkey, dog, cat, horse, hamster, gerbil, rabbit, ferret, horse, cattle, and sheep. An animal can be any mammal or bird.

The *B. hyodysenteriae* polypeptide analogues include those polypeptides having the amino acid sequence, wherein one or more of the amino acids are substituted with another amino acid which substitutions do not substantially alter the biological activity of the molecule.

According to the invention, the polypeptides of the invention produced recombinantly or by chemical synthesis and fragments or other derivatives or analogues thereof, including fusion proteins, may be used as an immunogen to generate antibodies that recognize the polypeptides.

A molecule is "antigenic" when it is capable of specifically interacting with an antigen recognition molecule of the immune system, such as an immunoglobulin (antibody) or T cell antigen receptor. An antigenic amino acid sequence contains at least about 5, and preferably at least about 10, amino acids. An antigenic portion of a molecule can be the portion that is immunodominant for antibody or T cell receptor recognition, or it can be a portion used to generate an antibody to the molecule by conjugating the antigenic portion to a carrier molecule for immunization. A molecule that is antigenic need not be itself immunogenic, *i*.*e*., capable of eliciting an immune response without a carrier.

An "antibody" is any immunoglobulin, including antibodies and fragments thereof, that binds a specific epitope. The term encompasses polyclonal, monoclonal, and chimeric antibodies, the last mentioned described in further detail in U.S. Patent Nos. 4,816,397 and 4,816,567, as well as antigen binding portions of antibodies, including Fab, F(ab')₂ and F(v) (including single chain antibodies). Accordingly, the phrase "antibody molecule" in its various grammatical forms as used herein contemplates both an intact immunoglobulin molecule and an immunologically active portion of an immunoglobulin molecule containing the antibody combining site. An "antibody combining site" is that structural portion of an antibody molecule comprised of heavy and light chain variable and hypervariable regions that specifically binds an antigen.

Exemplary antibody molecules are intact immunoglobulin molecules, substantially intact immunoglobulin molecules and those portions of an immunoglobulin molecule that contain the paratope, including those portions known in the art as Fab, Fab', F(ab')₂ and F(v), which portions are preferred for use in the therapeutic methods described herein.

Fab and F(ab')₂ portions of antibody molecules are prepared by the proteolytic reaction of papain and pepsin, respectively, on substantially intact antibody molecules by methods that are well-known. See for example, U.S. Patent No. 4,342,566 to Theofilopolous *et al*. Fab' antibody molecule portions are also well-known and are produced from F(ab')₂ portions followed by reduction with mercaptoethanol of the disulfide bonds linking the two heavy chain portions, and followed by alkylation of the resulting protein mercaptan with a reagent such as iodoacetamide. An antibody containing intact antibody molecules is preferred herein.

The phrase "monoclonal antibody" in its various grammatical forms refers to an antibody having only one species of antibody combining site capable of immunoreacting with a particular antigen. A monoclonal antibody thus typically displays a single binding affinity for any antigen with which it immunoreacts. A monoclonal antibody may therefore contain an antibody molecule having a plurality of antibody combining sites, each immunospecific for a different antigen; *e*.*g*., a bispecific (chimeric) monoclonal antibody.

The term "adjuvant" refers to a compound or mixture that enhances the immune response to an antigen. An adjuvant can serve as a tissue depot that slowly releases the antigen and also as a lymphoid system activator that non-specifically enhances the immune response [Hood et al., in Immunology, p. 384, Second Ed., Benjamin/Cummings, Menlo Park, California (1984)]. Often, a primary challenge with an antigen alone, in the absence of an adjuvant, will fail to elicit a humoral or cellular immune response. Adjuvants include, but are not limited to, complete Freund's adjuvant, incomplete Freund's adjuvant, saponin, mineral gels such as aluminium hydroxide, surface active substances such as lysolecithin, pluronic polyols, polyanions, peptides, oil or hydrocarbon emulsions, keyhole limpet hemocyanins, dinitrophenol, and potentially useful human adjuvants such as BCG (*bacille Calmette-Guerin*) and *Corynebacterium parvum.* Preferably, the adjuvant is pharmaceutically acceptable.

Various procedures known in the art may be used for the production of polyclonal antibodies to the polypeptides of the invention. For the production of antibody, various host animals can be immunised by injection with the polypeptide of the invention, including but not limited to rabbits, mice, rats, sheep, goats, *etc*. In one embodiment, a polypeptide of the invention can be conjugated to an immunogenic carrier, *e*.*g*., bovine serum albumin (BSA) or keyhole limpet hemocyanin (KLH). Various adjuvants may be used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (*bacille Calmette-Guerin*) and *Corynebacterium parvum.*

For preparation of monoclonal antibodies directed toward a polypeptide of the invention, any technique that provides for the production of antibody molecules by continuous cell lines in culture may be used. These include but are not limited to the hybridoma technique originally developed by Kohler et al., (1975) Nature, 256:495-497, the trioma technique, the human B-cell hybridoma technique [Kozbor et al., (1983) Immunology Today, 4:72], and the EBV-hybridoma technique to produce human monoclonal antibodies [Cole et al., (1985) in Monoclonal Antibodies and Cancer Therapy, pp. 77-96, Alan R. Liss, Inc.]. Immortal, antibody-producing cell lines can be created by techniques other than fusion, such as direct transformation of B lymphocytes with oncogenic DNA, or transfection with Epstein-Barr virus. *See, e.g.,* U.S. Patent Nos. 4,341,761; 4,399,121; 4,427,783; 4,444,887; 4,451,570; 4,466,917; 4,472,500; 4,491,632; and 4,493,890.

In an additional embodiment of the invention, monoclonal antibodies can be produced in germ-free animals utilising recent technology. According to the invention, chicken or swine antibodies may be used and can be obtained by using chicken or swine hybridomas or by transforming B cells with EBV virus *in vitro.* In fact, according to the invention, techniques developed for the production of "chimeric antibodies" [Morrison et al., (1984) J. Bacteriol., 159-870; Neuberger et al., (1984) Nature, 312:604-608; Takeda et al., (1985) Nature, 314:452-454] by splicing the genes from a mouse antibody molecule specific for a polypeptide of the invention together with genes from an antibody molecule of appropriate biological activity can be used; such antibodies are within the scope of this invention. Such chimeric antibodies are preferred for use in therapy of intestinal diseases or disorders (described *infra*), since the antibodies are much less likely than xenogenic antibodies to induce an immune response, in particular an allergic response, themselves.

According to the invention, techniques described for the production of single chain antibodies (U.S. Patent 4,946,778) can be adapted to produce single chain antibodies specific for a polypeptide of the invention. An additional embodiment of the invention utilises the techniques described for the construction of Fab expression libraries [Huse et al., (1989) Science, 246:1275-1281] to allow rapid and easy identification of monoclonal Fab fragments with the desired specificity for a polypeptide of the invention.

Antibody fragments, which contain the idiotype of the antibody molecule, can be generated by known techniques. For example, such fragments include but are not limited to: the F(ab')₂ fragment which can be produced by pepsin digestion of the antibody molecule; the Fab' fragments which can be generated by reducing the disulfide bridges of the F(ab')₂ fragment, and the Fab fragments which can be generated by treating the antibody molecule with papain and a reducing agent.

In the production of antibodies, screening for the desired antibody can be accomplished by techniques known in the art, *e*.*g*., radioimmunoassay, ELISA, "sandwich" immunoassays, immunoradiometric assays, gel diffusion precipitin reactions, immunodiffusion assays, *in situ* immunoassays (using colloidal gold, enzyme or radioisotope labels, for example), Western blots, precipitation reactions, agglutination assays (*e*.*g*., gel agglutination assays, hemagglutination assays), complement fixation assays, immunofluorescence assays, protein A assays, immunoelectrophoresis assays, *etc.* In one embodiment, antibody binding is detected by detecting a label on the primary antibody. In another embodiment, the primary antibody is detected by detecting binding of a secondary antibody or reagent to the primary antibody. In a further embodiment, the secondary antibody is labelled. Many means are known in the art for detecting binding in an immunoassay and are within the scope of the present invention. For example, to select antibodies that recognise a specific epitope of a polypeptide of the invention, one may assay generated hybridomas for a product that binds to a fragment of a polypeptide of the invention containing such epitope.

The invention also covers diagnostic and prognostic methods to detect the presence of *B. hyodysenteriae* using a polypeptide of the invention and/or antibodies which bind to the polypeptide of the invention and kits useful for diagnosis and prognosis of *B. hyodysenteriae* infections.

Diagnostic and prognostic methods will generally be conducted using a biological sample obtained from an animal, such as chicken or swine. A "sample" refers to an animal's tissue or fluid suspected of containing a *Brachyspira* species, such as *B. hyodysenteriae,* or its polynucleotides or its polypeptides. Examples of such tissue or fluids include, but not limited to, plasma, serum, faecal material, urine, lung, heart, skeletal muscle, stomach, intestines, and *in vitro* cell culture constituents.

The invention provides methods for detecting the presence of a polypeptide of the invention in a sample, with the following steps: (a) contacting a sample suspected of containing a polypeptide of the invention with an antibody (preferably bound to a solid support) that specifically binds to the polypeptide of the invention under conditions which allow for the formation of reaction complexes comprising the antibody and the polypeptide of the invention; and (b) detecting the formation of reaction complexes comprising the antibody and polypeptide of the invention in the sample, wherein detection of the formation of reaction complexes indicates the presence of the polypeptide of the invention in the sample.

Preferably, the antibody used in this method is derived from an affinity-purified polyclonal antibody, and more preferably a monoclonal antibody. In addition, it is preferable for the antibody molecules used herein be in the form of Fab, Fab', F(ab')₂ or F(v) portions or whole antibody molecules.

Particularly preferred methods for detecting *B. hyodysenteriae* based on the above method include enzyme linked immunosorbent assays, radioimmunoassays, immunoradiometric assays and immunoenzymatic assays, including sandwich assays using monoclonal and/or polyclonal antibodies.

Three such procedures that are especially useful utilise either polypeptide of the invention (or a fragment thereof) labelled with a detectable label, antibody Ab₁ labelled with a detectable label, or antibody Ab₂ labelled with a detectable label. The procedures may be summarized by the following equations wherein the asterisk indicates that the particle is labelled and "AA" stands for the polypeptide of the invention:
A. AA* + Ab₁ = AA*Ab₁
B. AA + Ab*₁ = AAAb₁*
C. AA + Ab₁ + Ab₂* = Ab₁ AA Ab₂*

The procedures and their application are all familiar to those skilled in the art and accordingly may be utilised within the scope of the present invention. The "competitive" procedure, Procedure A, is described in U.S. Patent Nos. 3,654,090 and 3,850,752. Procedure B is representative of well-known competitive assay techniques. Procedure C, the "sandwich" procedure, is described in U.S. Patent Nos. RE 31,006 and 4,016,043. Still other procedures are known, such as the "double antibody" or "DASP" procedure, and can be used.

In each instance, the polypeptide of the invention form complexes with one or more antibody(ies) or binding partners and one member of the complex is labelled with a detectable label. The fact that a complex has formed and, if desired, the amount thereof, can be determined by known methods applicable to the detection of labels.

It will be seen from the above, that a characteristic property of Ab₂ is that it will react with Ab₁. This reaction is because Ab₁, raised in one mammalian species, has been used in another species as an antigen to raise the antibody, Ab₂. For example, Ab₂ may be raised in goats using rabbit antibodies as antigens. Ab₂ therefore would be anti-rabbit antibody raised in goats. For purposes of this description and claims, Ab₁ will be referred to as a primary antibody, and Ab₂ will be referred to as a secondary or anti-Ab₁ antibody.

The labels most commonly employed for these studies are radioactive elements, enzymes, chemicals that fluoresce when exposed to ultraviolet light, and others. Examples of fluorescent materials capable of being utilised as labels include fluorescein, rhodamine and auramine. A particular detecting material is anti-rabbit antibody prepared in goats and conjugated with fluorescein through an isothiocyanate. Examples of preferred isotope include ³H, ¹⁴C, ³²P, ³⁵S, ³⁶Cl, ⁵¹Cr, ⁵⁷Co, ⁵⁸Co, ⁵⁹Fe, ⁹⁰Y, ¹³¹I, ¹³¹I, and ¹⁸⁶Re. The radioactive label can be detected by any of the currently available counting procedures. While many enzymes can be used, examples of preferred enzymes are peroxidase, β-glucuronidase, β-D-glucosidase, β-D-galactosidase, urease, glucose oxidase plus peroxidase and alkaline phosphatase. Enzymes are conjugated to the selected particle by reaction with bridging molecules such as carbodiimides, diisocyanates, glutaraldehyde and the like. Enzyme labels can be detected by any of the presently utilized colorimetric, spectrophotometric, fluorospectrophotometric, amperometric or gasometric techniques. U.S. Patent Nos. 3,654,090; 3,850,752; and 4,016,043 are referred to by way of example for their disclosure of alternate labelling material and methods.

The invention also provides a method of detecting antibodies to a polypeptide of the invention in biological samples, using the following steps: (a) providing a polypeptide of the invention or a fragment thereof; (b) incubating a biological sample with said polypeptide of the invention under conditions which allow for the formation of an antibody-antigen complex; and (c) determining whether an antibody-antigen complex with the polypeptide of the invention is formed.

In another embodiment of the invention there are provided *in vitro* methods for evaluating the level of antibodies to a polypeptide of the invention in a biological sample using the following steps: (a) detecting the formation of reaction complexes in a biological sample according to the method noted above; and (b) evaluating the amount of reaction complexes formed, which amount of reaction complexes corresponds to the level of polypeptide of the invention in the biological sample.

Further there are provided *in vitro* methods for monitoring therapeutic treatment of a disease associated with *B. hyodysenteriae* in an animal host by evaluating, as describe above, the levels of antibodies to a polypeptide of the invention in a series of biological samples obtained at different time points from an animal host undergoing such therapeutic treatment.

The present invention further provides methods for detecting the presence or absence *of B. hyodysenteriae* in a biological sample by: (a) bringing the biological sample into contact with a polynucleotide probe or primer of polynucleotide of the invention under suitable hybridizing conditions; and (b) detecting any duplex formed between the probe or primer and nucleic acid in the sample.

According to one embodiment of the invention, detection *of B. hyodysenteriae* may be accomplished by directly amplifying polynucleotide sequences from biological sample, using known techniques and then detecting the presence of polynucleotide of the invention sequences.

In one form of the invention, the target nucleic acid sequence is amplified by PCR and then detected using any of the specific methods mentioned above. Other useful diagnostic techniques for detecting the presence of polynucleotide sequences include, but are not limited to: 1) allele-specific PCR; 2) single stranded conformation analysis; 3) denaturing gradient gel electrophoresis; 4) RNase protection assays; 5) the use of proteins which recognize nucleotide mismatches, such as the *E. coli* mutS protein; 6) allele-specific oligonucleotides; and 7) fluorescent *in situ* hybridisation.

In addition to the above methods polynucleotide sequences may be detected using conventional probe technology. When probes are used to detect the presence of the desired polynucleotide sequences, the biological sample to be analysed, such as blood or serum, may be treated, if desired, to extract the nucleic acids. The sample polynucleotide sequences may be prepared in various ways to facilitate detection of the target sequence; *e*.*g*. denaturation, restriction digestion, electrophoresis or dot blotting. The targeted region of the sample polynucleotide sequence usually must be at least partially single-stranded to form hybrids with the targeting sequence of the probe. If the sequence is naturally single-stranded, denaturation will not be required. However, if the sequence is double-stranded, the sequence will probably need to be denatured. Denaturation can be carried out by various techniques known in the art.

Sample polynucleotide sequences and probes are incubated under conditions that promote stable hybrid formation of the target sequence in the probe with the putative desired polynucleotide sequence in the sample. Preferably, high stringency conditions are used in order to prevent false positives.

Detection, if any, of the resulting hybrid is usually accomplished by the use of labelled probes. Alternatively, the probe may be unlabeled, but may be detectable by specific binding with a ligand that is labelled, either directly or indirectly. Suitable labels and methods for labelling probes and ligands are known in the art, and include, for example, radioactive labels which may be incorporated by known methods (e.g., nick translation, random priming or kinasing), biotin, fluorescent groups, chemiluminescent groups (e.g., dioxetanes, particularly triggered dioxetanes), enzymes, antibodies and the like. Variations of this basic scheme are known in the art, and include those variations that facilitate separation of the hybrids to be detected from extraneous materials and/or that amplify the signal from the labelled moiety.

It is also contemplated within the scope of this invention that the nucleic acid probe assays of this invention may employ a cocktail of nucleic acid probes capable of detecting the desired polynucleotide sequences of this invention. Thus, in one example to detect the presence of polynucleotide sequences of this invention in a cell sample, more than one probe complementary to a polynucleotide sequences is employed and in particular the number of different probes is alternatively 2, 3, or 5 different nucleic acid probe sequences.

The polynucleotide sequences described herein (preferably in the form of probes) may also be immobilised to a solid phase support for the detection of *Brachyspira* species, including but not limited to *B. hyodysenteriae, B. intermedia, B. alvinipulli, B. aalborgi, B. innocens, B. murdochii, and B. pilosicoli.* Alternatively the polynucleotide sequences described herein will form part of a library of DNA molecules that may be used to detect simultaneously a number of different genes from *Brachyspira* species, such as *B. hyodysenteriae.* In a further alternate form of the invention polynucleotide sequences described herein together with other polynucleotide sequences (such as from other bacteria or viruses) may be immobilised on a solid support in such a manner permitting identification of the presence of a *Brachyspira* species, such as *B. hyodysenteriae* and/or any of the other polynucleotide sequences bound onto the solid support.

Techniques for producing immobilised libraries of DNA molecules have been described in the art. Generally, most prior art methods describe the synthesis of single-stranded nucleic acid molecule libraries, using for example masking techniques to build up various permutations of sequences at the various discrete positions on the solid substrate. U.S. Patent No. 5,837,832 describes an improved method for producing DNA arrays immobilised to silicon substrates based on very large scale integration technology. In particular, U.S. Patent No. 5,837,832 describes a strategy called "tiling" to synthesize specific sets of probes at spatially defined locations on a substrate that may be used to produce the immobilised DNA libraries of the present invention. U.S. Patent No. 5,837,832 also provides references for earlier techniques that may also be used. Thus polynucleotide sequence probes may be synthesised *in situ* on the surface of the substrate.

Alternatively, single-stranded molecules may be synthesised off the solid substrate and each pre-formed sequence applied to a discrete position on the solid substrate. For example, polynucleotide sequences may be printed directly onto the substrate using robotic devices equipped with either pins or pizo-electric devices.

The library sequences are typically immobilised onto or in discrete regions of a solid substrate. The substrate may be porous to allow immobilisation within the substrate or substantially non-porous, in which case the library sequences are typically immobilised on the surface of the substrate. The solid substrate may be made of any material to which polypeptides can bind, either directly or indirectly. Examples of suitable solid substrates include flat glass, silicon wafers, mica, ceramics and organic polymers such as plastics, including polystyrene and polymethacrylate. It may also be possible to use semi-permeable membranes such as nitrocellulose or nylon membranes, which are widely available. The semi-permeable membranes may be mounted on a more robust solid surface such as glass. The surfaces may optionally be coated with a layer of metal, such as gold, platinum or other transition metal.

Preferably, the solid substrate is generally a material having a rigid or semi-rigid surface. In preferred embodiments, at least one surface of the substrate will be substantially flat, although in some embodiments it may be desirable to physically separate synthesis regions for different polymers with, for example, raised regions or etched trenches. It is also preferred that the solid substrate is suitable for the high density application of DNA sequences in discrete areas of typically from 50 to 100 µm, giving a density of 10000 to 40000 dots/cm⁻².

The solid substrate is conveniently divided up into sections. This may be achieved by techniques such as photoetching, or by the application of hydrophobic inks, for example teflon-based inks (Cel-line, USA).

Discrete positions, in which each different member of the library is located may have any convenient shape, e.g., circular, rectangular, elliptical, wedge-shaped, etc.

Attachment of the polynucleotide sequences to the substrate may be by covalent or non-covalent means. The polynucleotide sequences may be attached to the substrate via a layer of molecules to which the library sequences bind. For example, the polynucleotide sequences may be labelled with biotin and the substrate coated with avidin and/or streptavidin. A convenient feature of using biotinylated polynucleotide sequences is that the efficiency of coupling to the solid substrate can be determined easily. Since the polynucleotide sequences may bind only poorly to some solid substrates, it is often necessary to provide a chemical interface between the solid substrate (such as in the case of glass) and the nucleic acid sequences. Examples of suitable chemical interfaces include hexaethylene glycol. Another example is the use of polylysine coated glass, the polylysine then being chemically modified using standard procedures to introduce an affinity ligand. Other methods for attaching molecules to the surfaces of solid substrate by the use of coupling agents are known in the art, see for example WO98/49557.

Binding of complementary polynucleotide sequences to the immobilised nucleic acid library may be determined by a variety of means such as changes in the optical characteristics of the bound polynucleotide sequence (*i*.*e*. by the use of ethidium bromide) or by the use of labelled nucleic acids, such as polypeptides labelled with fluorophores. Other detection techniques that do not require the use of labels include optical techniques such as optoacoustics, reflectometry, ellipsometry and surface plasmon resonance (see WO97/49989).

Thus, the present invention provides a solid substrate having immobilized thereon at least one polynucleotide of the present invention, preferably two or more different polynucleotide sequences of the present invention.

The present invention also can be used as a prophylactic or therapeutic, which may be utilised for the purpose of stimulating humoral and cell mediated responses in animals, such as chickens and swine, thereby providing protection against colonisation with *Brachyspira* species, including but not limited to *B. hyodysenteriae, B. intermedia, B. alvinipulli, B. aalborgi, B. innocens, B. murdochii, and B. pilosicoli.* Natural infection with a *Brachyspira* species, such as *B. hyodysenteriae* induces circulating antibody titres against the proteins described herein. Therefore, the amino acid sequences described herein or parts thereof, have the potential to form the basis of a systemically or orally administered prophylactic or therapeutic to provide protection against swine dysentery.

Accordingly, in one embodiment the present invention provides the amino acid sequences described herein or fragments thereof or antibodies that bind the amino acid sequences or the polynucleotide sequences described herein in a therapeutically effective amount admixed with a pharmaceutically acceptable carrier, diluent, or excipient.

The phrase "therapeutically effective amount" is used herein to mean an amount sufficient to reduce by at least about 15%, preferably by at least 50%, more preferably by at least 90%, and most preferably prevent, a clinically significant deficit in the activity, function and response of the animal host. Alternatively, a therapeutically effective amount is sufficient to cause an improvement in a clinically significant condition in the animal host.

The phrase "pharmaceutically acceptable" refers to molecular entities and compositions that are physiologically tolerable and do not typically produce an allergic or similarly untoward reaction, such as gastric upset and the like, when administered to an animal. The term "carrier" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Water or saline solutions and aqueous dextrose and glycerol solutions are preferably employed as carriers, particularly for injectable solutions. Suitable pharmaceutical carriers are described in Martin, *Remington's Pharmaceutical Sciences,* 18th Ed., Mack Publishing Co., Easton, PA, (1990).

In a more specific form of the invention there are provided pharmaceutical compositions comprising therapeutically effective amounts of the amino acid sequences described herein or an analogue, fragment or derivative product thereof or antibodies thereto together with pharmaceutically acceptable diluents, preservatives, solubilizes, emulsifiers, adjuvants and/or carriers. Such compositions include diluents of various buffer content (*e*.*g*., Tris-HCl, acetate, phosphate), pH and ionic strength and additives such as detergents and solubilizing agents (e.g., Tween 80, Polysorbate 80), anti-oxidants (*e*.*g*., ascorbic acid, sodium metabisulfite), preservatives (*e*.*g*., Thimersol, benzyl alcohol) and bulking substances (*e*.*g*., lactose, mannitol). The material may be incorporated into particulate preparations of polymeric compounds such as polylactic acid, polyglycolic acid, etc. or into liposomes. Hylauronic acid may also be used. Such compositions may influence the physical state, stability, rate of *in vivo* release, and rate of *in vivo* clearance of the present proteins and derivatives. *See, e*.*g*., Martin, Remington's Pharmaceutical Sciences, 18th Ed. (1990, Mack Publishing Co., Easton, PA 18042) pages 1435-1712 that are herein incorporated by reference. The compositions may be prepared in liquid form, or may be in dried powder, such as lyophilised form.

Alternatively, the polynucleotides of the invention can be optimized for expression in plants (*e*.*g*., corn). The plant may be transformed with plasmids containing the optimized polynucleotides. Then the plant is grown, and the proteins of the invention are expressed in the plant, or the plant-optimized version is expressed. The plant is later harvested, and the section of the plant containing the proteins of the invention is processed into feed for the animal. This animal feed will impart immunity against *B. hyodysenteriae* when eaten by the animal. Examples of prior art detailing these methods can be found in U.S. Patent 5,914,123 (Arntzen, et al.); U.S. Patent 6,034,298 (Lam, et al.); and U.S. Patent 6,136,320 (Arntzen, et al.).

It will be appreciated that pharmaceutical compositions provided accordingly to the invention may be administered by any means known in the art. Preferably, the pharmaceutical compositions for administration are administered by injection, orally, or by the pulmonary, or nasal route. The amino acid sequences described herein or antibodies derived therefrom are more preferably delivered by intravenous, intraarterial, intraperitoneal, intramuscular, or subcutaneous routes of administration. Alternatively, the amino acid sequence described herein or antibodies derived therefrom, properly formulated, can be administered by nasal or oral administration.

Also encompassed by the present invention is the use of polynucleotide sequences of the invention, as well as antisense and ribozyme polynucleotide sequences hybridisable to a polynucleotide sequence encoding an amino acid sequence according to the invention, for manufacture of a medicament for modulation of a disease associated *B. hyodysenteriae.*

Polynucleotide sequences encoding antisense constructs or ribozymes for use in therapeutic methods are desirably administered directly as a naked nucleic acid construct. Uptake of naked nucleic acid constructs by bacterial cells is enhanced by several known transfection techniques, for example those including the use of transfection agents. Examples of these agents include cationic agents (for example calcium phosphate and DEAE-dextran) and lipofectants. Typically, nucleic acid constructs are mixed with the transfection agent to produce a composition.

Alternatively the antisense construct or ribozymes may be combined with a pharmaceutically acceptable carrier or diluent to produce a pharmaceutical composition. Suitable carriers and diluents include isotonic saline solutions, for example phosphate-buffered saline. The composition may be formulated for parenteral, intramuscular, intravenous, subcutaneous, intraocular, oral or transdermal administration. The routes of administration described are intended only as a guide since a skilled practitioner will be able to determine readily the optimum route of administration and any dosage for any particular animal and condition.

The invention also includes kits for screening animals suspected of being infected with a *Brachyspira* species, such as *B. hyodysenteriae* or to confirm that an animal is infected with a *Brachyspira* species, such as *B. hyodysenteriae.* In a further embodiment of this invention, kits suitable for use by a specialist may be prepared to determine the presence or absence of *Brachyspira* species, including but not limited to *B. hyodysenteriae, B. intermedia, B. alvinipulli, B. aalborgi, B. innocens, B. murdochii, and B. pilosicoli* in suspected infected animals or to quantitatively measure a *Brachyspira* species, including but not limited to *B. hyodysenteriae, B. intermedia, B. alvinipulli, B. aalborgi and B. pilosicoli* infection. In accordance with the testing techniques discussed above, such kits can contain at least a labelled version of one of the amino acid sequences described herein or its binding partner, for instance an antibody specific thereto, and directions depending upon the method selected, e.g., "competitive," "sandwich," "DASP" and the like. Alternatively, such kits can contain at least a polynucleotide sequence complementary to a portion of one of the polynucleotide sequences described herein together with instructions for its use. The kits may also contain peripheral reagents such as buffers, stabilizers, *etc.*

Accordingly, a test kit for the demonstration of the presence of a *Brachyspira* species, including but not limited to *B. hyodysenteriae, B. intermedia, B. alvinipulli, B. aalborgi, B. innocens, B. murdochii, and B. pilosicoli,* may contain the following:
(a) a predetermined amount of at least one labelled immunochemically reactive component obtained by the direct or indirect attachment of one of the amino acid sequences described herein or a specific binding partner thereto, to a detectable label;
(b) other reagents; and
(c) directions for use of said kit.

More specifically, the diagnostic test kit may contain:
(a) a known amount of one of the amino acid sequences described herein as described above (or a binding partner) generally bound to a solid phase to form an immunosorbent, or in the alternative, bound to a suitable tag, or there are a plural of such end products, etc;
(b) if necessary, other reagents; and
(c) directions for use of said test kit.

In a further variation, the test kit may contain:
(a) a labelled component which has been obtained by coupling one of the amino acid sequences described herein to a detectable label;
(b) one or more additional immunochemical reagents of which at least one reagent is a ligand or an immobilized ligand, which ligand is selected from the group consisting of:
   (i) a ligand capable of binding with the labelled component (a);
   (ii) a ligand capable of binding with a binding partner of the labelled component (a);
   (iii) a ligand capable of binding with at least one of the component(s) to be determined; or
   (iv) a ligand capable of binding with at least one of the binding partners of at least one of the component(s) to be determined; and
(c) directions for the performance of a protocol for the detection and/or determination of one or more components of an immunochemical reaction between one of the amino acid sequences described herein and a specific binding partner thereto.

### Preparation of genomic library

A genomic library is prepared using an Australian porcine field isolate of *B. hyodysenteriae* (strain WA1). This strain has been well-characterised and shown to be virulent following experimental challenge of pigs. The cetyltrimethylammonium bromide (CTAB) method is used to prepare high quality chromosomal DNA suitable for preparation of genomic DNA libraries. *B. hyodysenteriae* is grown in 100 ml anaerobic trypticase soy broth culture to a cell density of 10⁹ cells/ml. The cells are harvested at 4,000 x *g* for 10 minutes, and the cell pellet resuspended in 9.5 ml TE buffer. SDS is added to a final concentration of 0.5 % (w/v), and the cells lysed at 37°C for 1 hour with 100 µg of Proteinase K. NaCl is added to a final concentration of 0.7 M and 1.5 ml CTAB/NaCl solution (10% w/v CTAB, 0.7 M NaCl) is added before incubating the solution at 65°C for 20 minutes. The lysate is extracted with an equal volume of chloroform/isoamyl alcohol, and the tube is centrifuged at 6,000 x *g* for 10 minutes to separate the phases. The aqueous phase is transferred to a fresh tube and 0.6 volumes of isopropanol are added to precipitate the high molecular weight DNA. The precipitated DNA is collected using a hooked glass rod and transferred to a tube containing 1 ml of 70 % (v/v) ethanol. The tube is centrifuged at 10,000 x *g* and the pelleted DNA redissolved in 4 ml TE buffer overnight. A cesium chloride gradient containing 1.05 g/ml CsCl and 0.5 mg/ml ethidium bromide is prepared using the redissolved DNA solution. The gradient is transferred to 4 ml sealable centrifuge tubes and centrifuged at 70,000 x *g* overnight at 15°C. The separated DNA is visualized under an ultraviolet light, and the high molecular weight DNA is withdrawn from the gradient using a 15-g needle. The ethidium bromide is removed from the DNA by sequential extraction with CsCl-saturated isopropanol. The purified chromosomal DNA is dialysed against 2 litres TE buffer and precipitated with isopropanol. The resuspended genomic DNA is sheared using a GeneMachines Hydroshear (Genomic Solutions, Ann Arbor, MI), and the sheared DNA is filled-in using Klenow DNA polymerase to generate blunt-end fragments. One hundred ng of the blunt-end DNA fragments is ligated with 25 ng of pSMART VC vector (Lucigen, Meddleton, WI) using CloneSmart DNA ligase. The ligated DNA is then electroporated into *E. coli* electrocompetent cells. A small insert (2-3 kb) library and medium insert (3-10 kb) library is constructed into the low copy version of the pSMART VC vector.

### Genomic sequencing

After the genomic library is obtained, individual clones of E. coli containing the pSMART VC vector are picked. The plasmid DNA is purified and sequenced. The purified plasmids are subjected to automated direct sequencing of the pSMART VC vector using the forward and reverse primers specific for the pSMART VC vector. Each sequencing reaction is performed in a 10 µl volume consisting of 200 ng of plasmid DNA, 2 pmol of primer, and 4 µl of the ABI PRISM™ BigDye Terminator Cycle Sequencing Ready Reaction Mix (PE Applied Biosystems, Foster City, CA). Cycling conditions involve a 2 minute denaturing step at 96°C, followed by 25 cycles of denaturation at 96°C for 10 seconds, and a combined primer annealing and extension step at 60°C for 4 minutes. Residual dye terminators are removed from the sequencing products by precipitation with 95% (v/v) ethanol containing 85 mM sodium acetate (pH 5.2), 3mM EDTA (pH 8), and vacuum dried. The plasmids are sequenced in duplicate using each primer. Sequencing products are analysed using an ABI 373A DNA Sequencer (PE Applied Biosystems).

### Annotation

Partial genome sequences for *B. hyodysenteriae* are assembled and annotated using a range of public domain bioinformatics tools to analyse and re-analyse the sequences as part of a quality assurance procedure on data analysis. Open reading frames (ORFs) are predicted using a variety of programs including GeneMark, GLIMMER, ORPHEUS, SELFID and GetORF. Putative ORFs are examined for homology (DNA and protein) with existing international databases using searches including BLAST and FASTA. All the predicted ORFs are analysed to determine their cellular localisation using programs including PSI-BLAST, FASTA, MOTIFS, FINDPATTERNS, PHD, SIGNALP and PSORT. Databases including Interpro, Prosite, ProDom, Pfam and Blocks are used to predict surface associated proteins such as transmembrane domains, leader peptides, homologies to known surface proteins, lipoprotein signature, outer membrane anchoring motifs and host cell binding domains. Phylogenetic and other molecular evolution analysis is conducted with the identified genes and with other species to assist in the assignment of function. The *in silico* analysis of both partially sequenced genomes produces a comprehensive list of all the predicted ORFs present in the sequence data available. Each ORF is interrogated for descriptive information such as predicted molecular weight, isoelectric point, hydrophobicity, and subcellular localisation to enable correlation with the *in vitro* properties of the native gene product. Predicted genes which encode proteins similar to surface localized components and/or virulence factors in other pathogenic bacteria are selected as potential vaccine targets.

### Bioinformatics Results

The shotgun sequencing of the *B. hyodysenteriae* genome results in 73% (2347.8 kb out of a predicted 2300 kb) of the genome being sequenced. The *B. hyodysenteriae* sequence is comprised of 171 contigs with an average contig size of 13.7 kb. For *B. hyodysenteriae,* 1860 open-reading frames (ORFs) are predicted from the 171 contigs. Comparison of the predicted ORFs with genes present in the nucleic acid and protein databases indicate that approximately 70% of the ORFs have homology with genes contained in the public databases. The remaining 30% of the predicted ORFs have no known identity.

### Vaccine candidates

To help reduce the number of ORFs that would be tested as a vaccine candidate, a two part test is established. Potential vaccine candidates are required to have some, albeit minor, homology with outer surface proteins and virulence factors present in the public databases. The potential vaccine candidate ORFs also have to be present in many strains of *B. hyodysenteriae*. Of the 1860 ORFs obtained in the genomic shotgun sequencing, many passed one or both prongs of this test but the results of only seven are presented here. Table 1 shows seven clones selected as potential vaccine targets and their similarity with other known amino acid sequences obtained from SWISS-PROT database. It is noted that the percent identity of amino acids does not rise above 46% while the percent similarity of amino acids remains less than 65%, thus indicating that these ORFs are unique.

**Table 1**

| Gene | Identity of Protein With Highest Homology | Identity (amino acids) | Similarity (amino acids) | Accession Number |
|---|---|---|---|---|
| NAV-H1 | surface antigen (BspA) of *Bacteroides forsythus* | 37% (60/158) | 53% (93/174) | AAC82625.1 |
| NAV-H2 | outer membrane protein (OmpA) family of *Leptospira interrogans* | 29% (130/445) | 47% (213/445) | NP_713795.1 |
| NAV-H3 | amino-acid ABC transporter extracellular binding protein | 34% (73/212) | 57% (122/212) | P54535 |
| NAV-H5 | transport system surface lipoprotein of *Desulfitobacterium hafniense* | 41% (141/339) | 59% (201/339) | ZP_00099372.1 |
| NAV-H6 | transport system surface lipoprotein of *Desulfitobacterium hafniense* | 46% (157/339) | 63% (214/339) | ZP_00099372.1 |
| NAV-H11 | Toxin A of *Clostridium difficile* | 24% (49/201) | 44% (89/201) | P16154 |
| NAV-H13 | oligopeptide binding protein (OppA) of *Salmonella typhimurium* | 35% (173/492) | 52% (258/492) | P06202 |

The DNA and amino acid sequences of NAV-H1 are found in SEQ ID NOs: I and 2, respectively. The DNA and amino acid sequences of NAV-H2 are found in SEQ ID NOs: 3 and 4, respectively. The DNA and amino acid sequences of NAV-H3 are found in SEQ ID NOs: 5 and 6, respectively. The DNA and amino acid sequences of NAV-H5 are found in SEQ ID NOs: 7 and 8, respectively. The DNA and amino acid sequences of NAV-H6 are found in SEQ ID NOs: 9 and 10, respectively. The DNA and amino acid sequences of NAV-H11 are found in SEQ ID NOs: 11 and 12, respectively. The DNA and amino acid sequences of NAV-H13 are found in SEQ ID NOs: 13 and 14, respectively.

### Analysis of gene distribution using polymerase chain reaction (PCR)

Two primer pairs which anneal to different regions of the target gene encoding region are designed and optimised for PCR detection. Individual primers are designed using Oligo Explorer 1.2 and primer sets with calculated melting temperatures of approximately 55-60°C are selected. These primers sets are also selected to generate PCR products greater than 200bp. A medium-stringency primer annealing temperature of 50°C is selected for the distribution analysis PCR. The medium-stringency conditions would allow potential minor mismatched sequences (because of strain differences) occurring at the primer binding sites to not affect primer binding. Distribution analysis of the seven *B. hyodysenteriae* target genes are performed on 23 strains of *B. hyodysenteriae*, including two strains which have been shown to be avirulent. Primer sets used in the distribution analysis are shown in Table 2. PCR analysis is performed in a 25 µl total volume using *Taq* DNA polymerase (Biotech International, Thurmont, MD). The amplification mixture consists of 1x PCR buffer (containing 1.5 mM of MgCl₂), 1 U of *Taq* DNA polymerase, 0.2 mM of each dNTP (Amersham Pharmacia Biotech, Piscataway, NJ), 0.5 µM of the primer pair, and 1 µl purified chromosomal template DNA. Cycling conditions involve an initial template denaturation step of 5 minutes at 94°C, follow by 35 cycles of denaturation at 94°C for 30 seconds, annealing at 50°C for 15 seconds, and primer extension at 68°C for 4 minutes. The PCR products are subjected to electrophoresis in 1% (w/v) agarose gels in 1x TAE buffer (40 mM Tris-acetate, 1 mM EDTA), staining with a 1 µg/ml ethidium bromide solution and viewing over UV light.

Six of the *B. hyodysenteriae* ORFs selected as vaccine candidates are present in each strain tested. One clone, NAV-H3, is present in 96% (22 out of 23) of the *B. hyodysenteriae* strains analyzed. The strain which did not possess the NAV-H3 ORF is not one of the avirulent strains.

**Table 2**

| Gene | Primer name | Primer Sequence (5'-3') |
|---|---|---|
| NAV-H1 | H1-F76 | ccattttctttagatgaatcatatgg (SEQ ID NO: 15) |
| | H1-R1008 | attaggcatagatttgccaaaaat (SEQ ID NO: 16) |
| | H1-F139 | attggtattgatgctgcagcttt (SEQ ID NO: 17) |
| | H1-R389 | ttagcgcaaccatcaaatacgt (SEQ ID NO: 18) |
| NAV-H2 | H2-F67 | ataacacatagattctattggaatttagaa (SEQ ID NO: 19) |
| | H2-R1209 | tcttaatttgattatgatatcaactcttct (SEQ ID NO: 20) |
| | H2-F498 | cgccaatgttgaaactaatagc (SEQ ID NO: 21) |
| | H2-R1069 | ctgattttgctcttttttctgataa (SEQ ID NO: 22) |
| NAV-H3 | H3-F52 | tgtcgtaattattcaaatacaaataactc (SEQ ID NO: 23) |
| | H3-R783 | taaatacttatctactaatttttgataagtgc (SEQ ID NO: 24) |
| | H3-F266 | tgcagtttgatgatcttattacagc (SEQ ID NO: 25) |
| | H3-R543 | gcctgcaatatcaaatctctctact (SEQ ID NO: 26) |
| NAV-H5 | H5-F55 | tgcagtaatacaaacagtggaaaatc (SEQ ID NO: 27) |
| | H5-R1071 | atttatataattaatttttactaaattcaaagg (SEQ ID NO: 28) |
| | H5-F256 | atgggagcaaaattgatagtaacac (SEQ ID NO: 29) |
| | H5-R847 | aagcagcaacagcattataaacag (SEQ ID NO: 30) |
| NAV-H6 | H6-F70 | ccaaatactaatacaagtacttttgaaatag (SEQ ID NO: 31) |
| | H6-R783 | ttttatataattaacctttgctgaattt (SEQ ID NO: 32) |
| | H6-F212 | ttgctcaccctgctgtagttag (SEQ ID NO: 33) |
| | H6-R554 | gcaacagcattataaacagattctc (SEQ ID NO: 34) |
| NAV-H11 | H11-F101 | gcttaggtttttcaggacattctcat (SEQ ID NO: 35) |
| | H11-R765 | tttctttaattcatcaactgtttcaa (SEQ ID NO: 36) |
| | H11-F265 | aaagatactgataaagaaatggggc (SEQ ID NO: 37) |
| | H11-R550 | ccttttttatataccaatcttcttcttcag (SEQ ID NO: 38) |
| NAV-H13 | H13-F286 | aaatggacagacggaaaaacag (SEQ ID NO: 39) |
| | H13-R1209 | attttctttccacatctgctga (SEQ ID NO: 40) |
| | H13-F518 | ctttccctacattttatccgctt (SEQ ID NO: 41) |
| | H13-R962 | ccgcctcttgtaatctgctc (SEQ ID NO: 42) |

The seven ORFs selected for further testing as potential vaccine candidates are next cloned into expression vectors, expressed, purified, and evaluated for immunogenicity.

### pTrcHis Plasmid Extraction

*Escherichia coli* JM109 clones harboring the pTrcHis plasmid (Invitrogen, Carlsbad, CA) are streaked out from glycerol stock storage onto Luria-Bertani (LB) agar plates supplemented with 100 mg/l ampicillin and incubated at 37°C for 16 hours. A single colony is used to inoculate 10 ml of LB broth supplemented with 100 mg/l ampicillin, and the broth culture is incubated at 37°C for 12 hours with shaking. The entire overnight culture is centrifuged at 5,000 x *g* for 10 minutes, and the plasmid contained in the cells is extracted using the QIAprep Spin Miniprep Kit (Qiagen, Doncaster VIC). The pelleted cells are resuspended with 250 µl cell resuspension buffer P1 and then are lysed with the addition of 250 µl cell lysis buffer P2. The lysed cells are neutralized with 350 µl neutralization buffer N3, and the precipitated cell debris is pelleted by centrifugation at 20,000 x *g* for 10 minutes. The supernatant is transferred to a spin column and centrifuged at 10,000 x *g* for 1 minute. After discarding the flow-through, 500 µl wash buffer PE is applied to the column and centrifuged as before. The flow-through is discarded, and the column is dried by centrifugation at 20,000 x *g* for 3 minutes. The plasmid DNA is eluted from the column with 100 µl elution buffer EB. The purified plasmid is quantified using a Dynaquant DNA fluorometer (Hoefer, San Francisco, CA), and the DNA concentration is adjusted to 100 µg/ml by dilution with TE buffer. The purified pTrcHis plasmid is stored at -20°C.

### Vector Preparation

Two µg of the purified pTrcHis plasmid is digested at 37°C for 1-4 hours in a total volume of 50 µl containing 5 U of *Eco*R1 (New England Biolabs, Beverly, MA) and 5 U of *Xho*1 (New England Biolabs) in 100 mM Tris-HCl (pH 7.5), 50 mM NaCl, 10 mM MgCl₂, 1 mM DTT and 100 µg/ml BSA. The restricted vector is verified by electrophoresing 1 µl of the digestion reaction through a 1% (w/v) agarose gel in 1X TAE buffer at 90V for 1 hour. The electrophoresed DNA is stained with 1 µg/ml ethidium bromide and is viewed over ultraviolet (UV) light.

Linearised pTrcHis vector is purified using the UltraClean PCR Clean-up Kit (Mo Bio Laboratories, Carlsbad, CA). Briefly, the restriction reaction (50 µl) is mixed with 250 µl SpinBind buffer B1, and the entire volume is added to a spin-column. After centrifugation at 8,000 x *g* for 1 minute, the flow-through is discarded and 300 µl SpinClean buffer B2 is added to the column. The column is centrifuged as before, and the flow-through is discarded before drying the column at 20,000 x *g* for 3 minutes. The purified vector is eluted from the column with 50 µl TE buffer. Purified linear vector is quantified using a fluorometer, and the DNA concentration is adjusted to 50 µg/ml by dilution with TE buffer. The purified restricted vector is stored at -20°C.

### Primer design for Insert Preparation

Primer pairs are designed to amplify as much of the coding region of the target gene as possible using genomic DNA as the starting point. All primers sequences include terminal restriction enzyme sites to enable cohesive-end ligation of the resultant amplicon into the linearised pTrcHis vector. The primer sequences used for cloning are shown in Table 3. The primers are tested using Amplify 1.2 (University of Wisconsin, Madison, WI) and the theoretical amplicon sequence is inserted into the appropriate position in the pTrcHis vector sequence. Deduced translation of the chimeric pTrcHis expression cassette is performed using Vector NTI version 6 (InforMax) to confirm that the gene inserts would be in the correct reading frame. Table 3 also provides the predicted molecular weight of the native protein in daltons and the apparent molecular weight of the expressed protein in kDa as determined from SDS-PAGE. The histidine-fusion of the recombinant protein adds approximately 4 kDa to the native protein.

**Table 3**

| Gene | Primer name | Primer Sequence (5'-3') | Predicted MW of native protein (Da) | Apparent MW of recombinant protein (kDa) |
|---|---|---|---|---|
| NAV-H1 | H1-F76-XhoI H1-R1008-EcoRI | gttctcgagccattttctttagatgaatcatatgg (SEQ ID NO: 43) gttgaattcattaggcatagatttgccaaaaat (SEQ ID NO: 44) | 37429 | 42.2 |
| NAV-H2 | H2-F57-XhoI H2-R1209-EcoRI | ctactcgagataacacatagattctattggaatttagaa (SEQ ID NO: 45) gttgaattctcttaatttgattatgatatcaactcttct (SEQ ID NO: 46) | 46934 | 49.7 |
| NAV-H3 | H3-F52-XhoI H3-R783-EcoRI | ctactcgagtgtcgtaattattcaaatacaaataactc (SEQ ID NO: 47) gttgaattctaaatacttatctactaatttttgataagtgc (SEQ ID NO: 48) | 29534 | 32.9 |
| NAV-H5 | H5-F55-XhoI H5-R1071-EcoRI | ctactcgagtgcagtaatacaaacagtggaaaatc (SEQ ID NO: 49) gttgaattcatttatataattaatttttactaaattcaaagg (SEQ ID NO: 50) | 39505 | 43.3 |
| NAV-H6 | H6-F70-XhoI H6-R783-EcoRI | agcctcgagccaaatactaatacaagtacttttgaaatag (SEQ ID NO: 51) gttgaattcttttatataattaacctttgctgaattt (SEQ ID NO: 52) | 39306 | 43.9 |
| NAV-H11 | H11-F4-XhoI H11-R843-EcoRI | ctactcgagaaatatatatctaatttgcatacacatac (SEQ ID NO: 53) gttgaattctacacttagtcctaatttatttatatcaa (SEQ ID NO: 54) | 32934 | 36.9 |
| NAV-H13 | H13-F52-XhoI H13-R1596-EcoRI | atactcgagtgttctaataataaatcagaagatgg (SEQ ID NO: 55) gttgaattcttctaaataacaatgatggaatttatg (SEQ ID NO: 56) | 60810 | 64.8 |

### Amplification of the gene inserts

Using genomic DNA, all target gene inserts are amplified by PCR in a 100 µl total volume using *Taq* DNA polymerase (Biotech International) and *Pfµ* DNA polymerase (Promega, Madison, WI). The amplification mixture consists of 1x PCR buffer (containing 1.5 mM of MgCl₂), 1U of *Taq* DNA polymerase, 0.01 U *Pfu* DNA polymerase, 0.2 mM of each dNTP (Amersham Pharmacia Biotech), 0.5 µM of the appropriate primer pair and 1 µl of purified chromosomal DNA. The chromosomal DNA is prepared from the same *B. hyodysenteriae* strain used for genome sequencing. Cycling conditions involve an initial template denaturation step of 5 minutes at 94°C, followed by 35 cycles of denaturation at 94°C for 30 seconds, annealing at 50°C for 15 seconds, and primer extension at 68°C for 4 minutes. The PCR products are subjected to electrophoresis in 1% (w/v) agarose gels in 1x TAE buffer, are stained with a 1 µg/ml ethidium bromide solution and are viewed over UV light. After verifying the presence of the correct size PCR product, the PCR reaction is purified using the UltraClean PCR Clean-up Kit (Mo Bio Laboratories, Carlsbad, CA). The PCR reaction (100 µl) is mixed with 500 µl SpinBind buffer B1, and the entire volume is added to a spin-column. After centrifugation at 8,000 x *g* for 1 minute, the flow-through is discarded, and 300 µl SpinClean buffer B2 is added to the column. The column is centrifuged as before and the flow-through is discarded before drying the column at 20,000 x *g* for 3 minutes. The purified vector is eluted from the column with 100 µl TE buffer.

### Restriction enzyme digestion of the gene inserts

Thirty µl of the purified PCR product is digested in a 50 µl total volume with 1 U of *Eco*R1 and 1 U of *Xho*1 in 100 mM Tris-HCl (pH 7.5), 50 mM NaCl, 10 mM MgCl₂, 1 mM DTT and 100 µg/ml BSA at 37°C for 1-4 hours. The digested insert DNA is purified using the UltraClean PCR Clean-up Kit (see above). Purified digested insert DNA is quantified using a fluorometer, and the DNA concentration is adjusted to 20 µg/ml by dilution with TE buffer. The purified restricted insert DNA is used immediately for vector ligation.

### Ligation of the gene inserts into the pTrcHis vector

Ligation reactions are all performed in a total volume of 20 µl. One hundred ng of *Xho*1/*Eco*R1-linearised pTrcHis is incubated with 20 ng of *Xho*1/*Eco*R1-restricted insert at 16°C for 16 hours in 30 mM Tris-HCl (pH 7.8), 10 mM MgCl₂, 10 mM DTT and 1 mM ATP containing 1 U of T4 DNA ligase (Promega). An identical ligation reaction containing no insert DNA is also included as a vector re-circularisation negative control.

### Transformation of pTrcHis ligations into E. coli cells

Competent *E. coli* JM109 (Promega) cells are thawed from -80°C storage on ice and then 50 µl of the cells are transferred into ice-cold 1.5 ml microfuge tubes containing 5 µl of the overnight ligation reactions (equivalent to 25 ng of pTrcHis vector). The tubes are mixed by gently tapping the bottom of each tube on the bench and left on ice for 30 minutes. The cells are then heat-shocked by placing the tubes into a 42°C waterbath for 45 seconds before returning the tube to ice for 2 minutes. The transformed cells are recovered in 1 ml LB broth for 1 hour at 37°C with gentle mixing. The recovered cells are harvested at 2,500 x *g* for 5 minutes, and the cells are resuspended in 50 µl of fresh LB broth. The entire 50 µl of resuspended cells are spread evenly onto a LB agar plate containing 100 mg/l ampicillin using a sterile glass rod. Plates are incubated at 37°C for 16 hours.

### Detection of recombinant pTrcHis constructs in E. coli by PCR

Twelve single transformant colonies for each construct are streaked onto fresh LB agar plates containing 100 mg/l ampicillin and incubated at 37°C for 16 hours. A single colony from each transformation event is resuspended in 50 µl of TE buffer and is boiled for 1 minute. Two µl of boiled cells are used as template for PCR. The amplification mixture consists of 1x PCR buffer (containing 1.5 mM of MgCl₂), 1 U of *Taq* DNA polymerase, 0.2 mM of each dNTP, 0.5 µM of the pTrcHis-F primer (5'-CAATTTATCAGACAATCTGTGTG-3' SEQ ID NO: 57) and 0.5 µM of the pTrcHis-R primer (5'-TGCCTGGCAGTTCCCTACTCTCG-3' SEQ ID NO: 58). Cycling conditions involve an initial template denaturation step of 5 minutes at 94°C, followed by 35 cycles of denaturation at 94°C for 30 seconds, annealing at 60°C for 15 seconds, and a primer extension at 72°C for 1 minute. The PCR products are subjected to electrophoresis in 1% (w/v) agarose gels in 1x TAE buffer, are stained with a 1 µg/ml ethidium bromide solution and are viewed over UV light. Cloning of the various inserts into the pTrcHis expression vector produces recombinant constructs of various sizes.

### Pilot expression of recombinant His-tagged proteins

Five to ten isolated colonies of recombinant pTrcHis construct in *E. coli* JM109 are inoculated into 3 ml LB broth in a 5 ml tube containing 100 mg/l ampicillin and 1 mM IPTG and incubated at 37°C for 16 hours with shaking. The cells are harvested by centrifugation at 5,000 x *g* for 10 minutes at 4°C. The supernatant is discarded, and each pellet is resuspended with 10 µl Ni-NTA denaturing lysis buffer (100 mM NaH₂PO₄, 10 mM Tris-HCl, 8 M urea, pH 8.0). After vortexing the tube for 1 minute, the cellular debris is pelleted by centrifugation at 10,000 x *g* for 10 minute at 4°C. The supernatant is transferred to a new tube and stored at -20°C until analysis.

### Sodium dodecyl sulfate polyarylamide gel electrophoresis (SDS-PAGE)

SDS-PAGE analysis of protein is performed using a discontinuous Tris-glycine buffer system. Thirty µl of protein sample are mixed with 10 µl of 4x sample treatment buffer (250 mM Tris-HCl (pH 6.0), 8% (w/v) SDS, 200 mM DTT, 40% (v/v) glycerol and 0.04 % (w/v) bromophenol blue). Samples are boiled for 5 minutes immediately prior to loading 10 µl of the sample into wells in the gel. The gel comprises a stacking gel (125 mM Tris-HCl ph 6.8, 4% w/v acylamide, 0.15% w/v bis-acrylamide and 0.1% w/v SDS) and a separating gel (375 mM Tris-HCl ph 8.8, 12% w/v acylamide, 0.31 % w/v bis-acrylamide and 0.1 % w/v SDS). These gels are polymerised by the addition of 0.1% (v/v) TEMED and 0.05% (w/v) freshly prepared ammonium sulphate solution and cast into the mini-Protean dual slab cell (Bio-Rad, Hercules, California). Samples are run at 150 V at room temperature (RT) until the bromophenol blue dye reaches the bottom of the gel. Pre-stained molecular weight standards are electrophoresed in parallel with the samples in order to allow molecular weight estimations. After electrophoresis, the gel is immediately stained using Coomassie Brilliant Blue G250 (Bio-Rad) or is subjected to electro-transfer onto nitrocellulose membrane for Western blotting.

### Western Blot Analysis

Electrophoretic transfer of separated proteins from the SDS-PAGE gel to nitrocellulose membrane is performed using the Towbin transfer buffer system. After electrophoresis, the gel is equilibrated in transfer buffer (25 mM Tris, 192 mM glycine, 20% v/v methanol, pH 8.3) for 15 minutes. The proteins in the gel are electro-transferred to nitrocellulose membrane (Protran, Schleicher and Schuell BioScience, Inc., Keene, NH) using the mini-Protean transblot apparatus (Bio-Rad) at 30 V overnight at 4°C. The freshly transferred nitrocellulose membrane containing the separated proteins is blocked with 10 ml of tris-buffered saline (TBS) containing 5% (w/v) skim milk powder for 1 hour at RT. The membrane is washed with TBS containing 0.1% (v/v) Tween 20 (TBST) and then is incubated with 10 mL mouse anti-his antibody (diluted 5,000-fold with TBST) for 1 hour at RT. After washing three times for 5 minutes with TBST, the membrane is incubated with 10 mL goat anti-mouse IgG (whole molecule)-AP diluted 5,000-fold in TBST for 1 hour at RT. The membrane is developed using the Alkaline Phosphatase Substrate Kit (Bio-Rad). The development reaction is stopped by washing the membrane with distilled water. The membrane is then dried and scanned for presentation.

### Verification of reading frame of the recombinant pTrcHis constructs by direct sequence analysis

Two transformant clones for each construct which produced the correct sized PCR products are inoculated into 10 ml LB broth containing 100 mg/l ampicillin and incubated at 37°C for 12 hours with shaking. The entire overnight cultures are centrifuged at 5,000 x g for 10 minutes, and the plasmid contained in the cells are extracted using the QIAprep Spin Miniprep Kit as described previously. The purified plasmid is quantified using a fluorometer. Both purified plasmids are subjected to automated direct sequencing of the pTrcHis expression cassette using the pTrcHis-F and pTrcHis-R primers. Each sequencing reaction is performed in a 10 µl volume consisting of 200 ng of plasmid DNA, 2 pmol of primer, and 4 µl of the ABI PRISM™ BigDye Terminator Cycle Sequencing Ready Reaction Mix (PE Applied Biosystems, Foster City, CA). Cycling conditions involve a 2 minute denaturing step at 96°C, followed by 25 cycles of denaturation at 96°C for 10 seconds, and a combined primer annealing and extension step at 60°C for 4 minutes. Residual dye terminators are removed from the sequencing products by precipitation with 95% (v/v) ethanol containing 85 mM sodium acetate (pH 5.2), 3mM EDTA (pH 8), and vacuum dried. The plasmids are sequenced in duplicate using each primer. Sequencing products are analysed using an ABI 373A DNA Sequencer (PE Applied Biosystems). Nucleotide sequencing of the pTrcHis constructs verifies that the expression cassette is in the correct reading frame for all the constructs. The predicted translation of the pTrcHis expression cassette indicates that all the recombinant his-tagged proteins and the deduced amino acid sequence of the native *B. hyodysenteriae* proteins are identical.

### Expression and purification of recombinant His-tagged proteins

A single colony of the recombinant pTrcHis construct in *E. coli* JM109 is inoculated into 50 ml LB broth in a 250 ml conical flask containing 100 mg/l ampicillin and incubated at 37°C for 16 hours with shaking. A 2 l conical flask containing 1 l of LB broth supplemented with 100 mg/l ampicillin is inoculated with 10 ml of the overnight culture and incubated at 37°C until the optical density of the cells at 600 nm is 0.5 (approximately 3-4 hours). The culture is then induced by adding IPTG to a final concentration of 1 mM, and the cells are returned to 37°C with shaking. After 5 hours of induction, the culture is transferred to 250 ml centrifuge bottles, and the bottles are centrifuged at 5,000 x g for 20 minutes at 4°C. The supernatant is discarded, and each pellet is resuspended with 8 ml Ni-NTA denaturing lysis buffer (100 mM NaH₂PO₄, 10 mM Tris-HCl, 8 M urea, pH 8.0). The resuspended cells are stored at -20°C overnight.

The cell suspension is removed from -20°C storage and thawed on ice. The cell lysate is then sonicated on ice 3 times for 30 seconds with 1 minute incubation on ice between sonication rounds. The lysed cells are cleared by centrifugation at 20,000 x g for 10 minutes at 4°C, and the supernatant is transferred to a 15 ml column containing a 0.5 ml bed volume of Ni-NTA agarose resin (Qiagen). The recombinant His₆-tagged protein is allowed to bind to the resin for 1 hour at 4°C with end-over-end mixing. The resin is then washed with 30 ml of Ni-NTA denaturing wash buffer (100 mM NaH₂PO₄, 10 mM Tris-HCl, 8 M urea, pH 6.3) before elution with 12 ml of Ni-NTA denaturing elution buffer (100 mM NaH₂PO₄, 10 mM Tris-HCl, 8 M urea, pH 4.5). Four 3 ml fractions of the eluate are collected and stored at 4°C. Thirty µl of each eluate is treated with 10 µl of 4x sample treatment buffer and boiled for 5 minutes. The samples are subjected to SDS-PAGE and stained with Coomassie Brilliant Blue G250 (Bio-Rad). The stained gel is equilibrated in distilled water for 1 hour and dried between two sheets of cellulose overnight at RT.

Expression of the selected recombinant *E. coli* clones is performed in medium-scale to generate sufficient recombinant protein for vaccination of mice (see below). All recombinant proteins possessing the hexa-histidine fusion (4 kDa) produce a major protein with an apparent molecular weight similar to the predicted molecular weight of the native protein (see Table 3). These proteins are highly reactive in Western blotting using the anti-His antibody. Purification of the his-tagged recombinant proteins by affinity chromatography under denaturing conditions is successful. SDS-PAGE and Coomassie blue staining of all recombinant proteins show that a purification of at least 85% is achieved. Recombinant protein yields of 2 mg/L, are consistently obtained using this expression protocol.

### Dialysis and lyophilisation of the purified recombinant His-tagped protein

The eluted proteins are pooled and transferred into a hydrated dialysis tube (Spectrum Laboratories, Inc., Los Angeles, CA) with a molecular weight cut-off (MWCO) of 3,500 Da. A 200 µl aliquot of the pooled eluate is taken and quantified using a commercial Protein Assay (Bio-Rad). The proteins are dialysed against 21 of distilled water at 4°C with stirring. The dialysis buffer is changed 8 times at 12-hourly intervals. The dialysed proteins are transferred from the dialysis tube into a 50 ml centrifuge tubes (40 ml maximum volume), and the tubes are placed at -80°C overnight. Tubes are placed into a MAXI freeze-drier (Heto-Holten, Allerod, Denmark) and lyophilised to dryness. The lyophilised proteins are then re-hydrated with PBS to a calculated concentration of 2 mg/ml and stored at -20°C. Following dialysis and lyophilisation, stable recombinant antigen is successfully produced.

### Serology using purified recombinant protein

Twenty µg of purified recombinant protein is loaded into a 7 cm IEF well, electrophoresed through a 10% (w/v) SDS-PAGE gel, and electro-transferred to nitrocellulose membrane. The membrane is blocked with TBS-skim milk (5% w/v) and assembled into the multi-screen apparatus (Bio-Rad). The wells are incubated with 100 µl of diluted pig serum (100-fold) for 1 hour at RT. The pig serum was obtained from high health status pigs (n=3), experimentally challenged pigs showing clinical SD (n=5), naturally infected seroconverting pigs (n=5), and pigs recovered from natural infection (n=4). The membrane then is removed from the apparatus and washed three times with TBST (0.1% v/v) before incubating with 10 ml of goat anti-swine IgG (whole molecule)-AP (5,000-fold) for 1 hour at RT. The membrane is washed three times with TBST before color development using an Alkaline Phosphatase Substrate Kit (Bio-Rad). The membrane is washed with tap water when sufficient development has occurred, dried and scanned for presentation.

All the proteins cloned are recognized by 80-100% of the panel of pig serum, regardless of health status, thus indicating that the genes had been expressed *in vivo* and that the pigs were able to induce a systemic immune response following exposure to the spirochaete.

### Vaccination of mice using the purified recombinant his-tagged proteins

For each of the purified recombinant his-tagged proteins, ten mice are systemically and orally immunized to determine whether the recombinant protein would be immunogenic. The recombinant protein is emulsified with 30% (v/v) water in oil adjuvant and injected intramuscularly into the quadraceps muscle of ten mice (Balb/cJ: 5 weeks old males). All mice receive 100 µg of protein in a total volume of 100 µl. Three weeks after the first vaccination, all mice receive a second intramuscular vaccination identical to the first vaccination. All mice are killed two weeks after the second vaccination. Sera are obtained from the heart at post-mortem and tested in Western blot analysis for antibodies against cellular extracts of *B. hyodysenteriae.*

### Western blot analysis

Twenty µg of purified recombinant protein is loaded into a 7 cm IEF well, electrophoresed through a 10% (w/v) SDS-PAGE gel, and electro-transferred to nitrocellulose membrane. The membrane is blocked with TBS-skim milk (5% w/v) and assembled into the multi-screen apparatus (Bio-Rad). The wells are incubated with 100 µl of diluted mouse serum (100-fold) for 1 hour at RT. The membrane is removed from the apparatus and washed three times with TBST (0.1 % v/v) before incubating with 10 ml of goat anti-mouse IgG (whole molecule)-AP (5,000-fold) for 1 hour at RT. The membrane is washed three times with TBST before color development using an Alkaline Phosphatase Substrate Kit (Bio-Rad). The membrane is washed with tap water when sufficient development has occurred, dried and scanned for presentation.

Western blot analysis shows a significant increase in antibody reactivity in the mice towards the recombinant vaccine antigens following vaccination. All the mice recognize recombinant proteins which are similar in molecular weight to that of the Coomassie blue stained purified recombinant proteins. These experiments provide evidence that the recombinant proteins are immunogenic when used to vaccinate mice, and that the vaccination protocol employed can induce specific circulating antibody titres against the antigen.

### Vaccination of pigs using the purified recombinant his-tagged proteins

For each of the purified recombinant his-tagged proteins, ten sero-negative pigs are injected intramuscularly with 1 mg of the particular antigen in 1 ml vaccine volume. The antigen is emulsified with an equal volume of a water-in-oil adjuvant. The pigs are vaccinated at three weeks of age and again at six weeks of age. A second group of ten sero-negative pigs is used as negative controls and are left unvaccinated. All pigs are challenged with 100 ml of an active *B. hyodysenteriae* culture (∼10⁹ cells/ml) at eight weeks of age, and the pigs are observed for clinical signed of swine dysentery during the experiment (up to six weeks post-challenge) and at post-mortem examination.

### Diagnostic kit

Serum is obtained from pigs in a piggery with known infection of *B. hyodysenteriae,* from pigs known not to have been infected with *B. hyodysenteriae,* and from pigs in piggery with unknown infection status with *B. hyodysenteriae.* Twenty µg of purified recombinant protein is loaded into a 7 cm IEF well, electrophoresed through a 10% (w/v) SDS-PAGE gel, and electro-transferred to nitrocellulose membrane. The membrane is blocked with TBS-skim milk (5% w/v) and assembled into the multi-screen apparatus (Bio-Rad). The wells are incubated with 100 µl of diluted pig serum (100-fold) for 1 hour at RT. The membrane then is removed from the apparatus and washed three times with TBST (0.1% v/v) before incubating with 10 ml of goat anti-swine IgG (whole molecule)-AP (5,000-fold) for 1 hour at RT. The membrane is washed three times with TBST before color development using an Alkaline Phosphatase Substrate Kit (Bio-Rad). The membrane is washed with tap water when sufficient development has occurred, dried and scanned for presentation. The sera from the pigs known to be infected with *B. hyodysenteriae* (positive controls) reacts with the recombinant proteins while the sera from the pigs without infection (negative controls) do not react. One can determine if pigs are infected with *B. hyodysenteriae* by comparing the results to the positive and negative controls.

While this invention has been described with a reference to specific embodiments, it will be obvious to those of ordinary skill in the art that variations in these methods and compositions may be used and that it is intended that the invention may be practiced otherwise than as specifically described herein. Accordingly this invention includes all modifications encompassed within the spirit and scope of the invention as defined by the claims.

## Claims

1. A polynucleotide comprising the sequence selected from the group consisting of SEQ ID NO: 1, 3, 5, 7, 9, 11 and 13.

2. A plasmid comprising the polynucleotide of Claim 1.

3. The plasmid of Claim 2 wherein said plasmid is an expression vector.

4. A cell containing the plasmid of Claim 2.

5. A cell containing the plasmid of Claim 3.

6. An immunogenic composition comprising the plasmid of Claim 3.

7. A vaccine composition for the treatment or prevention of *Brachyspira hyodysenteriae* comprising the expression vector of Claim 3.

8. A vaccine composition for the treatment or prevention of *Brachyspira hyodysenteriae* comprising the cell of Claim 4.

9. A DNA molecule comprising a sequence that is at least 70% homologous to the polynucleotide of Claim 1.

10. A plasmid comprising the polynucleotide of Claim 9.

11. The DNA molecule of Claim 9 wherein said DNA molecule is at least 80% homologous to the polynucleotide of Claim 1.

12. A plasmid comprising the polynucleotide of Claim 11.

13. The DNA molecule of Claim 9 wherein said DNA molecule is at least 90% homologous to the polynucleotide of Claim 1.

14. A plasmid comprising the polynucleotide of Claim 13.

15. A polypeptide comprising the sequence selected from the group consisting of SEQ ID NOs: 2, 4, 6, 8, 10, 12, and 14.

16. A polynucleotide comprising a sequence which encodes the polypeptide of Claim 15.

17. A plasmid comprising the polynucleotide of Claim 16.

18. The plasmid of Claim 17 wherein said plasmid is an expression vector.

19. A cell containing the plasmid of Claim 17.

20. An immunogenic composition comprising the cell of Claim 19.

21. A protein comprising a sequence that is at least 70% homologous to the polypeptide of Claim 15.

22. The protein of Claim 21 wherein said protein is at least 80% homologous to the polypeptide of Claim 15.

23. The protein of Claim 22 wherein said protein is at least 90% homologous to the polypeptide of Claim 15.

24. An immunogenic composition comprising the protein of Claim 23.

25. An immunogenic composition comprising the polypeptide of Claim 15.

26. A vaccine composition for the treatment or prevention of *Brachyspira hyodysenteriae* comprising the polypeptide of Claim 15.

27. A monoclonal antibody that binds to the polypeptide of Claim 15.

28. A kit for the diagnosis of presence of *Brachyspira hyodysenteriae* in an animal, said kit comprising the monoclonal antibody of Claim 27.

29. A kit for the diagnosis of presence of *Brachyspira hyodysenteriae* in an animal, said kit comprising the polypeptides of Claim 15.

30. A kit for the diagnosis of presence of *Brachyspira hyodysenteriae* in an animal, said kit comprising the polynucleotides of Claim 1.

31. A method of generating an immune response to *Brachyspira hyodysenteriae* in an animal comprising administering to said animal the immunogenic composition of Claim 24.

32. A method of generating an immune response to *Brachyspira hyodysenteriae* in an animal comprising administering to said animal the immunogenic composition of Claim 25.

33. A method of generating an immune response to *Brachyspira hyodysenteriae* in an animal comprising administering to said animal the immunogenic composition of Claim 6.

34. A method of generating an immune response to *Brachyspira hyodysenteriae* in an animal comprising administering to said animal the immunogenic composition of Claim 20.

35. A method of treating or preventing a disease caused by *Brachyspira hyodysenteriae* in an animal in need of said treatment comprising administering to said animal a therapeutically effective amount of the vaccine composition of Claim 7.

36. A method of treating or preventing a disease caused by *Brachyspira hyodysenteriae* in an animal in need of said treatment comprising administering to said animal a therapeutically effective amount of the vaccine composition of Claim 8.

37. A method of treating or preventing a disease caused by *Brachyspira hyodysenteriae* in an animal in need of said treatment comprising administering to said animal a therapeutically effective amount of the vaccine composition of Claim 26.


SEQUENCE LISTING

<110> Hampson, David
      La, Tom
      Bellgard, Matthew
      Murdoch University
      Novartis AG

<120> Novel Genes and Proteins of Brachyspira
  hyodysenteriae and Use of Same for Diagnosis and Therapy

<130> VA/H - 34260

<150> 2005902418
<151> 2005-05-12

<160> 58

<170> FastSEQ for Windows Version 4.0

<210> 1
<211> 1008
<212> DNA
<213> Brachyspira hyodysenteriae

<400> 1
atgttcataa agttaaaatt attattatta ttacttatac tattaatatc atgcaataat 60
tataaagtta cagatccatt ttctttagat gaatcatatg gcagcagtaa tatccctgtt 120
tctaatatac cggaaaatgt agactcaaaa tattttataa aatcagatta taaagaagat 180
aaaatagaat caataatgca ggaatatttt aacgaatata attgctatat aatcttttta 240
aaagacacta aagacaatat agataacaat aatataataa gcaccatcaa tacaatcatt 300
aaaaaagata aatatcttaa aggtgtttct cttgatttaa gcagaacaga tatgacaaaa 360
ttagcagata attcatttac tgataataaa aatttaattc atgtcaaatt acctaatact 420
ataacaacta taggtgtaag tgcattttca ggttgtttag ttttaaaaac aattaatttt 480
cctgcttcta taacagaaat ttcacaagag gcattcaaaa gctgcagatc tttatatttg 540
gcagacattt caaaaacaaa aatgacaata ataaataatg gaatatttaa tgactgtgct 600
agtttaatta ctattatgct tcctgaaact ataatagacg gtataaattc aaaggcattc 660
tatgactgtg catcattaaa aacaattaat atcccatcaa aaacaaaaat aattggagat 720

gctgtattct ataactgtaa atcattggaa tctataagat taaatgctag tataacagga 780
atgggagata gagttttttc aggatgcgtt gctttaaaaa atatagaata tgcaggaaat 840
aaatcttcag atataacatc cgtaggaata gatatatttg atgctcaatt aaccgcaaaa 900
aatttatatc taccaaatgt agcatcagat gacggaagct ggaataattt cttgggatat 960
gactggaaaa ataaaggcag catcattttt ggcaaatcta tgcctaat 1008

<210> 2
<211> 336
<212> PRT
<213> Brachyspira hyodysenteriae

<400> 2
Met Phe Ile Lys Leu Lys Leu Leu Leu Leu Leu Leu Ile Leu Leu Ile
1               5                   10                  15
Ser Cys Asn Asn Tyr Lys Val Thr Asp Pro Phe Ser Leu Asp Glu Ser
            20                  25                  30
Tyr Gly Ser Ser Asn Ile Pro Val Ser Asn Ile Pro Glu Asn Val Asp
        35                  40                  45
Ser Lys Tyr Phe Ile Lys Ser Asp Tyr Lys Glu Asp Lys Ile Glu Ser
    50                  55                  60
Ile Met Gln Glu Tyr Phe Asn Glu Tyr Asn Cys Tyr Ile Ile Phe Leu
65                  70                  75                  80
Lys Asp Thr Lys Asp Asn Ile Asp Asn Asn Asn Ile Ile Ser Thr Ile
                85                  90                  95
Asn Thr Ile Ile Lys Lys Asp Lys Tyr Leu Lys Gly Val Ser Leu Asp
            100                 105                 110
Leu Ser Arg Thr Asp Met Thr Lys Leu Ala Asp Asn Ser Phe Thr Asp
        115                 120                 125
Asn Lys Asn Leu Ile His Val Lys Leu Pro Asn Thr Ile Thr Thr Ile
    130                 135                 140
Gly Val Ser Ala Phe Ser Gly Cys Leu Val Leu Lys Thr Ile Asn Phe
145                 150                 155                 160
Pro Ala Ser Ile Thr Glu Ile Ser Gln Glu Ala Phe Lys Ser Cys Arg
                165                 170                 175
Ser Leu Tyr Leu Ala Asp Ile Ser Lys Thr Lys Met Thr Ile Ile Asn
            180                 185                 190
Asn Gly Ile Phe Asn Asp Cys Ala Ser Leu Ile Thr Ile Met Leu Pro
        195                 200                 205
Glu Thr Ile Ile Asp Gly Ile Asn Ser Lys Ala Phe Tyr Asp Cys Ala
    210                 215                 220
Ser Leu Lys Thr Ile Asn Ile Pro Ser Lys Thr Lys Ile Ile Gly Asp

225                 230                 235                 240
Ala Val Phe Tyr Asn Cys Lys Ser Leu Glu Ser Ile Arg Leu Asn Ala
                245                 250                 255
Ser Ile Thr Gly Met Gly Asp Arg Val Phe Ser Gly Cys Val Ala Leu
            260                 265                 270
Lys Asn Ile Glu Tyr Ala Gly Asn Lys Ser Ser Asp Ile Thr Ser Val
        275                 280                 285
Gly Ile Asp Ile Phe Asp Ala Gln Leu Thr Ala Lys Asn Leu Tyr Leu
    290                 295                 300
Pro Asn Val Ala Ser Asp Asp Gly Ser Trp Asn Asn Phe Leu Gly Tyr
305                 310                 315                 320
Asp Trp Lys Asn Lys Gly Ser Ile Ile Phe Gly Lys Ser Met Pro Asn
                325                 330                 335

<210> 3
<211> 1209
<212> DNA
<213> Brachyspira hyodysenteriae

<400> 3
atgaaaaaaa ttagtttatt tgtttttata ttttctactt tgctttttag tattttacat 60
tcagaaataa cacatagatt ctattggaat ttagaaaaag gaaaaagaat agaatctgta 120
aaaactgctg atgtagagta ttatgaaaat ggtatattaa caagcacata caaagaaaga 180
aatattgttg atttaactgt tattgctata gctccaaaag gcggttatag agtaagcgga 240
gtattcaaaa tctttagaat gatgaaaggt gagaaggttt ttcatcttga tgaagaatat 300
acaagtgatt ttattattca tactaatgga aaatttgaag ttccttataa ttattttatg 360
cctaatgtaa gacatgttcc tacttttcct gataatgagg taaagcttac tgaatcttgg 420
aatagagaat ctattgaaat agttaaagta aataatgccc ctaatttaac aatggcattg 480
tcatctgatt atctattcgc caatgttgaa actaatagcg acggaagcag aaatgctgtt 540
atacaatatc atataatgac tgataaagat ttacttcagg caggactttc aagaaaaggt 600
tatcctgaaa gaatatatgg ctttaattat ggtacatttc tttgggatat ggaaaaaaat 660
attcctgtat ctcagcagga gagatatcaa atattattcg gatatggaaa agaattgtct 720
tatgctagtt tgcagtataa gatgaatatt atcagtactt ataaaattta tgatacaata 780
acaaaagaag aagaagatta taatagaaaa aaacttgaag ataatctcta caatgatgat 840
aatgttacta tagatacagt accggaaggt ttggtattaa gacttggaga gattcttttt 900
gatactgatt cttatacttt aaaacctgaa gcaaaaaata ctatagataa tgttattaag 960
gcaataaaag aaacttatcc tgatagagaa attatagtag agggacatac tgataataca 1020
ggaagaaaag aatataatca ggacttatca gaaaaaagag caaaatcagt tgcagattat 1080
atcttgccta atcttgatca tgataaatta tcttataaag gttttgcaga tgatgaacct 1140
atagcttcta acgatactcc agacggaaga agaaaaaata gaagagttga tatcataatc 1200

aaattaaga                                                          1209

<210> 4
<211> 403
<212> PRT
<213> Brachyspira hyodysenteriae

<400> 4
Met Lys Lys Ile Ser Leu Phe Val Phe Ile Phe Ser Thr Leu Leu Phe
1               5                   10                  15
Ser Ile Leu His Ser Glu Ile Thr His Arg Phe Tyr Trp Asn Leu Glu
            20                  25                  30
Lys Gly Lys Arg Ile Glu Ser Val Lys Thr Ala Asp Val Glu Tyr Tyr
        35                  40                  45
Glu Asn Gly Ile Leu Thr Ser Thr Tyr Lys Glu Arg Asn Ile Val Asp
    50                  55                  60
Leu Thr Val Ile Ala Ile Ala Pro Lys Gly Gly Tyr Arg Val Ser Gly
65                  70                  75                  80
Val Phe Lys Ile Phe Arg Met Met Lys Gly Glu Lys Val Phe His Leu
                85                  90                  95
Asp Glu Glu Tyr Thr Ser Asp Phe Ile Ile His Thr Asn Gly Lys Phe
            100                 105                 110
Glu Val Pro Tyr Asn Tyr Phe Met Pro Asn Val Arg His Val Pro Thr
        115                 120                 125
Phe Pro Asp Asn Glu Val Lys Leu Thr Glu Ser Trp Asn Arg Glu Ser
    130                 135                 140
Ile Glu Ile Val Lys Val Asn Asn Ala Pro Asn Leu Thr Met Ala Leu
145                 150                 155                 160
Ser Ser Asp Tyr Leu Phe Ala Asn Val Glu Thr Asn Ser Asp Gly Ser
                165                 170                 175
Arg Asn Ala Val Ile Gln Tyr His Ile Met Thr Asp Lys Asp Leu Leu
            180                 185                 190
Gln Ala Gly Leu Ser Arg Lys Gly Tyr Pro Glu Arg Ile Tyr Gly Phe
        195                 200                 205
Asn Tyr Gly Thr Phe Leu Trp Asp Met Glu Lys Asn Ile Pro Val Ser
    210                 215                 220
Gln Gln Glu Arg Tyr Gln Ile Leu Phe Gly Tyr Gly Lys Glu Leu Ser
225                 230                 235                 240
Tyr Ala Ser Leu Gln Tyr Lys Met Asn Ile Ile Ser Thr Tyr Lys Ile
                245                 250                 255
Tyr Asp Thr Ile Thr Lys Glu Glu Glu Asp Tyr Asn Arg Lys Lys Leu

            260                 265                 270
Glu Asp Asn Leu Tyr Asn Asp Asp Asn Val Thr Ile Asp Thr Val Pro
        275                 280                 285
Glu Gly Leu Val Leu Arg Leu Gly Glu Ile Leu Phe Asp Thr Asp Ser
    290                 295                 300
Tyr Thr Leu Lys Pro Glu Ala Lys Asn Thr Ile Asp Asn Val Ile Lys
305                 310                 315                 320
Ala Ile Lys Glu Thr Tyr Pro Asp Arg Glu Ile Ile Val Glu Gly His
                325                 330                 335
Thr Asp Asn Thr Gly Arg Lys Glu Tyr Asn Gln Asp Leu Ser Glu Lys
            340                 345                 350
Arg Ala Lys Ser Val Ala Asp Tyr Ile Leu Pro Asn Leu Asp His Asp
        355                 360                 365
Lys Leu Ser Tyr Lys Gly Phe Ala Asp Asp Glu Pro Ile Ala Ser Asn
    370                 375                 380
Asp Thr Pro Asp Gly Arg Arg Lys Asn Arg Arg Val Asp Ile Ile Ile
385                 390                 395                 400
Lys Leu Arg

<210> 5
<211> 783
<212> DNA
<213> Brachyspira hyodysenteriae

<400> 5
atgaaaaaga ttattatttt tatactattt agtttaataa gttttacttc ctgtcgtaat 60
tattcaaata caaataactc agaaaatatt gtaactaatg aaatacaaat gaataataat 120
tattctaatg aaacattaag agttggaata tatatttatg attatcctat gcttcattta 180
agtaatgata atgtaggcgg atttgattat gatttaatga aaaaaatagc tgatatgtca 240
ggttttaaaa tacaatttat tccaatgcag tttgatgatc ttattacagc acttcgaata 300
aaggatatag atattattat agctgctatg agcataacag aagaaagaaa aaaacttgta 360
aatttttctg acagctatct aacagctggt caaagtattg tagtaaataa agaaaataca 420
aatataataa caactaatga tttaataggg aaaactgtag gagctataaa aaatactgtt 480
tctgatctta cagcatcaaa aatagaaggt attcataaag tagagagatt tgatattgca 540
ggcagtgctt ttctttcttt aaaaacaggt aaaatagatg ctatggtagt tgataaactt 600
acctgtatta actatcttca atatgataaa gatttaaaaa tagtcgaaac tatagaattc 660
ccagaaattg gctatggtat agctgtaaga aaagaggata atattttact aaataaaatt 720
aatagcggac ttaaagaaat aaaggataat ggcacttatc aaaaattagt agataagtat 780
tta 783

<210> 6
<211> 261
<212> PRT
<213> Brachyspira hyodysenteriae

<400> 6
Met Lys Lys Ile Ile Ile Phe Ile Leu Phe Ser Leu Ile Ser Phe Thr
1               5                   10                  15
Ser Cys Arg Asn Tyr Ser Asn Thr Asn Asn Ser Glu Asn Ile Val Thr
            20                  25                  30
Asn Glu Ile Gln Met Asn Asn Asn Tyr Ser Asn Glu Thr Leu Arg Val
        35                  40                  45
Gly Ile Tyr Ile Tyr Asp Tyr Pro Met Leu His Leu Ser Asn Asp Asn
    50                  55                  60
Val Gly Gly Phe Asp Tyr Asp Leu Met Lys Lys Ile Ala Asp Met Ser
65                  70                  75                  80
Gly Phe Lys Ile Gln Phe Ile Pro Met Gln Phe Asp Asp Leu Ile Thr
                85                  90                  95
Ala Leu Arg Ile Lys Asp Ile Asp Ile Ile Ile Ala Ala Met Ser Ile
            100                 105                 110
Thr Glu Glu Arg Lys Lys Leu Val Asn Phe Ser Asp Ser Tyr Leu Thr
        115                 120                 125
Ala Gly Gln Ser Ile Val Val Asn Lys Glu Asn Thr Asn Ile Ile Thr
    130                 135                 140
Thr Asn Asp Leu Ile Gly Lys Thr Val Gly Ala Ile Lys Asn Thr Val
145                 150                 155                 160
Ser Asp Leu Thr Ala Ser Lys Ile Glu Gly Ile His Lys Val Glu Arg
                165                 170                 175
Phe Asp Ile Ala Gly Ser Ala Phe Leu Ser Leu Lys Thr Gly Lys Ile
            180                 185                 190
Asp Ala Met Val Val Asp Lys Leu Thr Cys Ile Asn Tyr Leu Gln Tyr
        195                 200                 205
Asp Lys Asp Leu Lys Ile Val Glu Thr Ile Glu Phe Pro Glu Ile Gly
    210                 215                 220
Tyr Gly Ile Ala Val Arg Lys Glu Asp Asn Ile Leu Leu Asn Lys Ile
225                 230                 235                 240
Asn Ser Gly Leu Lys Glu Ile Lys Asp Asn Gly Thr Tyr Gln Lys Leu
                245                 250                 255
Val Asp Lys Tyr Leu
            260

<210> 7
<211> 1071
<212> DNA
<213> Brachyspira hyodysenteriae

<400> 7
atgaaaaaac ttcttactct tcttattgtg tttgtttttt tatacactgt ttcctgcagt 60
aatacaaaca gtggaaaatc acttggttat gaaatagcag ttatcatgag aaatatagat 120
gacaaatcat ttaatcaaag tacttggaga ggagtaaagg attatgctga acattatggc 180
ataagctata aatattataa agttcctgat aaaaatgtac aatcaacatt aaatgctata 240
gatattgctg ttcgtatggg agcaaaattg atagtaacac caggaagtat atttgaacct 300
gctatttata aagcacagga tatatatact aatgtacatt ttatattgat agacggaaag 360
cctcaggatg gaacttacac agattataag acagcaaaaa atactattgc tattctttat 420
gctgaagagg aagcaggatt tttagcagga tacgctatag taaaagaagg ttatactaat 480
ttaggagtta taggaggtat ggctcttcct cctgttataa aattcggata cggatttgta 540
caaggggcag aatttgcagc agatgaatta aaaattccta aagatagtat aaatataaaa 600
tacacttatg ttggaaatta tgatgattct caggaaaatc aaaatttagc tgcatcttgg 660
tataaaagcg gtacacaggt aatattcgct ccggcaggag gcgcttctta ttctgttata 720
aatgctgctg agaataatgg aggtttagtt gtaggaattg atgttgatca aagttttgaa 780
tctcctactg ttattacttc ttctatgaaa ttgataagag aatctgttta taatgctgtt 840
gctgcttatt ataatggtaa tttcaatgga ggaaaaactt tcatattaga tacaagagtt 900
aatggagtag gacttccaat gtctacttct aaatttaaag tttttaaaga atatgattat 960
aatatcatat ataactctct tatgagaaaa aaaattaggg tattaaaaga tactgatgcg 1020
gaaagtgttg ataaattacc tttgaattta gtaaaaatta attatataaa t 1071

<210> 8
<211> 357
<212> PRT
<213> Brachyspira hyodysenteriae

<400> 8
Met Lys Lys Leu Leu Thr Leu Leu Ile Val Phe Val Phe Leu Tyr Thr
1               5                   10                  15
Val Ser Cys Ser Asn Thr Asn Ser Gly Lys Ser Leu Gly Tyr Glu Ile
            20                  25                  30
Ala Val Ile Met Arg Asn Ile Asp Asp Lys Ser Phe Asn Gln Ser Thr
        35                  40                  45
Trp Arg Gly Val Lys Asp Tyr Ala Glu His Tyr Gly Ile Ser Tyr Lys
    50                  55                  60

Tyr Tyr Lys Val Pro Asp Lys Asn Val Gln Ser Thr Leu Asn Ala Ile
65                  70                  75                  80
Asp Ile Ala Val Arg Met Gly Ala Lys Leu Ile Val Thr Pro Gly Ser
                85                  90                  95
Ile Phe Glu Pro Ala Ile Tyr Lys Ala Gln Asp Ile Tyr Thr Asn Val
            100                 105                 110
His Phe Ile Leu Ile Asp Gly Lys Pro Gln Asp Gly Thr Tyr Thr Asp
        115                 120                 125
Tyr Lys Thr Ala Lys Asn Thr Ile Ala Ile Leu Tyr Ala Glu Glu Glu
    130                 135                 140
Ala Gly Phe Leu Ala Gly Tyr Ala Ile Val Lys Glu Gly Tyr Thr Asn
145                 150                 155                 160
Leu Gly Val Ile Gly Gly Met Ala Leu Pro Pro Val Ile Lys Phe Gly
                165                 170                 175
Tyr Gly Phe Val Gln Gly Ala Glu Phe Ala Ala Asp Glu Leu Lys Ile
            180                 185                 190
Pro Lys Asp Ser Ile Asn Ile Lys Tyr Thr Tyr Val Gly Asn Tyr Asp
        195                 200                 205
Asp Ser Gln Glu Asn Gln Asn Leu Ala Ala Ser Trp Tyr Lys Ser Gly
    210                 215                 220
Thr Gln Val Ile Phe Ala Pro Ala Gly Gly Ala Ser Tyr Ser Val Ile
225                 230                 235                 240
Asn Ala Ala Glu Asn Asn Gly Gly Leu Val Val Gly Ile Asp Val Asp
                245                 250                 255
Gln Ser Phe Glu Ser Pro Thr Val Ile Thr Ser Ser Met Lys Leu Ile
            260                 265                 270
Arg Glu Ser Val Tyr Asn Ala Val Ala Ala Tyr Tyr Asn Gly Asn Phe
        275                 280                 285
Asn Gly Gly Lys Thr Phe Ile Leu Asp Thr Arg Val Asn Gly Val Gly
    290                 295                 300
Leu Pro Met Ser Thr Ser Lys Phe Lys Val Phe Lys Glu Tyr Asp Tyr
305                 310                 315                 320
Asn Ile Ile Tyr Asn Ser Leu Met Arg Lys Lys Ile Arg Val Leu Lys
                325                 330                 335
Asp Thr Asp Ala Glu Ser Val Asp Lys Leu Pro Leu Asn Leu Val Lys
            340                 345                 350
Ile Asn Tyr Ile Asn
        355

<210> 9

<211> 1080
<212> DNA
<213> Brachyspira hyodysenteriae

<400> 9
atgattaaaa aaattatgtt atgtgtgtta tttatcacat ctttatttgc attgtcatgc 60
agcaaaagcc caaatactaa tacaagtact tttgaaatag ctgtacttat agacttaggt 120
cctattgatg ataaatcttt taatcaggga tcatgggaag gtgtaaagga ttatgccgaa 180
aattatggta taagttataa atattataga gttcctgata aggacataga ttcttattta 240
aacactatag atcttgcagt taaatgcggt gctaaattaa tagtaacccc tggatatatg 300
tttgaacctg caatatataa agctcaagat atttacacaa acgtaaattt catactgata 360
gatggagaac ctcaagacgg aacttataca gattataaaa catcacctaa tacagtagca 420
attctttatt ctgaagagga agcaggattt ttagcaggat acgctatagt aaaagaaggt 480
tatactaatt taggatttat gggaggtgtt gctcaccctg ctgtagttag attcggatac 540
ggatttgtac agggtgctga ttatgctgct gttgaatcaa aattacctaa agattctgtt 600
aatataaaat atacttatat aggcaattat gatcctacac ctgaaaatca aactttagct 660
acttcttggt ataaaagcgg agtacaagta atatttgctg cagctggtgc tgccggaaat 720
tcagttatga gtgctgctga aaataataac ggattggtaa taggagttga tgttgatcag 780
agttttgaat ctcctactgt tattacttct gctatgaaat tgataagaga atctgtttat 840
aatgctgttg catcttatta taacggaaat ttcagcggag gaaaaactac tatattagga 900
gctcaagtta aaggtatagg acttcctatg tctacttcta aatttaaagt ttttacagaa 960
tctgactata atattatata tgactctctt gtaacaaaaa caattaaagt attaaaagat 1020
actgatatag aaaatgtaaa taatttacat ttaaattcag caaaggttaa ttatataaaa 1080

<210> 10
<211> 360
<212> PRT
<213> Brachyspira hyodysenteriae

<400> 10
Met Ile Lys Lys Ile Met Leu Cys Val Leu Phe Ile Thr Ser Leu Phe
1               5                   10                  15
Ala Leu Ser Cys Ser Lys Ser Pro Asn Thr Asn Thr Ser Thr Phe Glu
            20                  25                  30
Ile Ala Val Leu Ile Asp Leu Gly Pro Ile Asp Asp Lys Ser Phe Asn
        35                  40                  45
Gln Gly Ser Trp Glu Gly Val Lys Asp Tyr Ala Glu Asn Tyr Gly Ile
    50                  55                  60
Ser Tyr Lys Tyr Tyr Arg Val Pro Asp Lys Asp Ile Asp Ser Tyr Leu
65                  70                  75                  80

Asn Thr Ile Asp Leu Ala Val Lys Cys Gly Ala Lys Leu Ile Val Thr
                85                  90                  95
Pro Gly Tyr Met Phe Glu Pro Ala Ile Tyr Lys Ala Gln Asp Ile Tyr
            100                 105                 110
Thr Asn Val Asn Phe Ile Leu Ile Asp Gly Glu Pro Gln Asp Gly Thr
        115                 120                 125
Tyr Thr Asp Tyr Lys Thr Ser Pro Asn Thr Val Ala Ile Leu Tyr Ser
    130                 135                 140
Glu Glu Glu Ala Gly Phe Leu Ala Gly Tyr Ala Ile Val Lys Glu Gly
145                 150                 155                 160
Tyr Thr Asn Leu Gly Phe Met Gly Gly Val Ala His Pro Ala Val Val
                165                 170                 175
Arg Phe Gly Tyr Gly Phe Val Gln Gly Ala Asp Tyr Ala Ala Val Glu
            180                 185                 190
Ser Lys Leu Pro Lys Asp Ser Val Asn Ile Lys Tyr Thr Tyr Ile Gly
        195                 200                 205
Asn Tyr Asp Pro Thr Pro Glu Asn Gln Thr Leu Ala Thr Ser Trp Tyr
    210                 215                 220
Lys Ser Gly Val Gln Val Ile Phe Ala Ala Ala Gly Ala Ala Gly Asn
225                 230                 235                 240
Ser Val Met Ser Ala Ala Glu Asn Asn Asn Gly Leu Val Ile Gly Val
                245                 250                 255
Asp Val Asp Gln Ser Phe Glu Ser Pro Thr Val Ile Thr Ser Ala Met
            260                 265                 270
Lys Leu Ile Arg Glu Ser Val Tyr Asn Ala Val Ala Ser Tyr Tyr Asn
        275                 280                 285
Gly Asn Phe Ser Gly Gly Lys Thr Thr Ile Leu Gly Ala Gln Val Lys
    290                 295                 300
Gly Ile Gly Leu Pro Met Ser Thr Ser Lys Phe Lys Val Phe Thr Glu
305                 310                 315                 320
Ser Asp Tyr Asn Ile Ile Tyr Asp Ser Leu Val Thr Lys Thr Ile Lys
                325                 330                 335
Val Leu Lys Asp Thr Asp Ile Glu Asn Val Asn Asn Leu His Leu Asn
            340                 345                 350
Ser Ala Lys Val Asn Tyr Ile Lys
        355                 360

<210> 11
<211> 843
<212> DNA

<213> Brachyspira hyodysenteriae

<400> 11
atgaaatata tatctaattt gcatacacat actaaatatt gcgatggtaa aaatacagtt 60
gaagaaaata tacaatatgc tatagataaa gaattaataa gcttaggttt ttcaggacat 120
tctcattttt ataaagatga tacttctatg tctgaagaaa acactattaa atatttagag 180
aatataaaaa aggcaaagga tatttataaa gataaaatac aggtatattt aggtatagaa 240
ggtgattatt attcaaattt aaataaagat actgataaag aaatggggct tgattataga 300
ataggctcag ttcattatat tgatgatggt aataattctt actttcctat agatatgtca 360
agagatactt ttaatgaaac tataaagcat tttggtgata tcaaagaagt tatttttaga 420
tattatgata atgttataaa aatgatagaa acacaaaaac ctgatattat aggtcatttg 480
gatttggtta gaaaatataa tttgaataaa gaatatttta ctgaagaaga agattggtat 540
ataaaaaagg tagatgaggt tatagatatt attgaaaaaa atggctccat agttgaaata 600
aatactaaac ttatgaataa aaataatttg gatgctcatt atcctaataa aaatactata 660
aaaaaattgt tagaaaaaaa tgtatctttt acaataaatt cagatgcaca tcaatgtaat 720
aatatagata attgttattt tgaaacagtt gatgaattaa agaaacttgg tgttaaatct 780
ataaaaatgt taatagacaa tagttttaaa gatattgata taaataaatt aggactaagt 840
gta 843

<210> 12
<211> 281
<212> PRT
<213> Brachyspira hyodysenteriae

<400> 12
Met Lys Tyr Ile Ser Asn Leu His Thr His Thr Lys Tyr Cys Asp Gly
1               5                   10                  15
Lys Asn Thr Val Glu Glu Asn Ile Gln Tyr Ala Ile Asp Lys Glu Leu
            20                  25                  30
Ile Ser Leu Gly Phe Ser Gly His Ser His Phe Tyr Lys Asp Asp Thr
        35                  40                  45
Ser Met Ser Glu Glu Asn Thr Ile Lys Tyr Leu Glu Asn Ile Lys Lys
    50                  55                  60
Ala Lys Asp Ile Tyr Lys Asp Lys Ile Gln Val Tyr Leu Gly Ile Glu
65                  70                  75                  80
Gly Asp Tyr Tyr Ser Asn Leu Asn Lys Asp Thr Asp Lys Glu Met Gly
                85                  90                  95
Leu Asp Tyr Arg Ile Gly Ser Val His Tyr Ile Asp Asp Gly Asn Asn
            100                 105                 110
Ser Tyr Phe Pro Ile Asp Met Ser Arg Asp Thr Phe Asn Glu Thr Ile
        115                 120                 125

Lys His Phe Gly Asp Ile Lys Glu Val Ile Phe Arg Tyr Tyr Asp Asn
    130                 135                 140

Val Ile Lys Met Ile Glu Thr Gln Lys Pro Asp Ile Ile Gly His Leu
145                 150                 155                 160

Asp Leu Val Arg Lys Tyr Asn Leu Asn Lys Glu Tyr Phe Thr Glu Glu
                165                 170                 175

Glu Asp Trp Tyr Ile Lys Lys Val Asp Glu Val Ile Asp Ile Ile Glu
            180                 185                 190

Lys Asn Gly Ser Ile Val Glu Ile Asn Thr Lys Leu Met Asn Lys Asn
        195                 200                 205

Asn Leu Asp Ala His Tyr Pro Asn Lys Asn Thr Ile Lys Lys Leu Leu
    210                 215                 220

Glu Lys Asn Val Ser Phe Thr Ile Asn Ser Asp Ala His Gln Cys Asn
225                 230                 235                 240

Asn Ile Asp Asn Cys Tyr Phe Glu Thr Val Asp Glu Leu Lys Lys Leu
                245                 250                 255

Gly Val Lys Ser Ile Lys Met Leu Ile Asp Asn Ser Phe Lys Asp Ile
            260                 265                 270

Asp Ile Asn Lys Leu Gly Leu Ser Val
        275                 280

<210> 13
<211> 1596
<212> DNA
<213> Brachyspira hyodysenteriae

<400> 13
atgaaaaaaa tatttatttt atttatgatt tttatatcat ttataatatc atgttctaat 60
aataaatcag aagatggtat atctatattt attaatacag gacctgaacc aaacactata 120
gatcctagta taaatgtaac ttcagatgca atattttatt tgatgcatac atttgaggga 180
ttgctagaga aggatatgaa tggaaaattg attccaggag ttgctgagag ttgggagata 240
agcgaagacg gacttactta tacattcaag cttagaacta attctaaatg gacagacgga 300
aaaacagttg ttgctggtga ttttgtatat tcttggcaaa gagttgttga tcctgctaca 360
ggcagtcaat atggatatca gcatgagcct gtaaaaaatg ctaaagcaat aactgcagga 420
gatatgccta aagaaagttt aggtataaaa gcaatagatg attatacttt ggaagttcag 480
ttggaagctc ctacagcata ttttttagaa cttttaactt tccctacatt ttatccgctt 540
agaaaggata ttatagaaca atacggagat gaatggactt taaatgctga tacttatata 600
ggaaacggag ctttcaaact tatagaaaga aacagagatg aaagtcttgt aatggtaaaa 660
aatactaatt attggaatat agaagatata gttcctgata aaataacatt tgttcttatg 720
gaaaatgaaa ctgcttctgt tgcaggtgtc aaagctggtt ctttgcattt tgcaagatct 780

ttcccaagac aggatataaa gacacttcaa aatgaaggac ttatagtaat aaagcctaga 840
atatcatcat attattactg cttaaattta actaatgaaa tattaaaaga tgtaagagta 900
agaagagcat tatctcttgc tattgacaga aattatatag tagagcagat tacaagaggc 960
ggagagaaac ctgcgggggc tttagttcct ttcggaatat cagactatga aggagatttc 1020
agagaaaacg gaggagaata tatagatata actaaagacg gatatgttaa gaatgtagaa 1080
gaagctaaaa aattgatggc tgaggcagga tatcctaatg gagaaggttt tcctgttatg 1140
gagtttaaag cagatcctgg attacatgta aaaatatttg aagctgttca gcagatgtgg 1200
aaagaaaatc ttggtattga tgtaacttta actcaggaag aatgggctgt atttttgcag 1260
acaagatatg atagaaatat aactatggct aggggagctt ggaatggaga ttttgatgat 1320
cctgtaaact ttatggcttt atgtttgagt tattctccta ataattacag tgtttatagt 1380
aatagggctt atgatgatat gataaatgag gttatgcttt caggagatca gaaattcagg 1440
atgcagacta tgcataaagc agaggaaatg ctaatgagag aagaagctat aatacccata 1500
tattattata cagaaccttt acttgtatct cctaaattaa aagatgttta ttatgattct 1560
ctaggatttc ataaattcca tcattgttat ttagaa 1596

<210> 14
<211> 532
<212> PRT
<213> Brachyspira hyodysenteriae

<400> 14
Met Lys Lys Ile Phe Ile Leu Phe Met Ile Phe Ile Ser Phe Ile Ile
1               5                   10                  15
Ser Cys Ser Asn Asn Lys Ser Glu Asp Gly Ile Ser Ile Phe Ile Asn
            20                  25                  30
Thr Gly Pro Glu Pro Asn Thr Ile Asp Pro Ser Ile Asn Val Thr Ser
        35                  40                  45
Asp Ala Ile Phe Tyr Leu Met His Thr Phe Glu Gly Leu Leu Glu Lys
    50                  55                  60
Asp Met Asn Gly Lys Leu Ile Pro Gly Val Ala Glu Ser Trp Glu Ile
65                  70                  75                  80
Ser Glu Asp Gly Leu Thr Tyr Thr Phe Lys Leu Arg Thr Asn Ser Lys
                85                  90                  95
Trp Thr Asp Gly Lys Thr Val Val Ala Gly Asp Phe Val Tyr Ser Trp
            100                 105                 110
Gln Arg Val Val Asp Pro Ala Thr Gly Ser Gln Tyr Gly Tyr Gln His
        115                 120                 125
Glu Pro Val Lys Asn Ala Lys Ala Ile Thr Ala Gly Asp Met Pro Lys
    130                 135                 140
Glu Ser Leu Gly Ile Lys Ala Ile Asp Asp Tyr Thr Leu Glu Val Gln
145                 150                 155                 160

Leu Glu Ala Pro Thr Ala Tyr Phe Leu Glu Leu Leu Thr Phe Pro Thr
                165                 170                 175
Phe Tyr Pro Leu Arg Lys Asp Ile Ile Glu Gln Tyr Gly Asp Glu Trp
            180                 185                 190
Thr Leu Asn Ala Asp Thr Tyr Ile Gly Asn Gly Ala Phe Lys Leu Ile
        195                 200                 205
Glu Arg Asn Arg Asp Glu Ser Leu Val Met Val Lys Asn Thr Asn Tyr
    210                 215                 220
Trp Asn Ile Glu Asp Ile Val Pro Asp Lys Ile Thr Phe Val Leu Met
225                 230                 235                 240
Glu Asn Glu Thr Ala Ser Val Ala Gly Val Lys Ala Gly Ser Leu His
                245                 250                 255
Phe Ala Arg Ser Phe Pro Arg Gln Asp Ile Lys Thr Leu Gln Asn Glu
            260                 265                 270
Gly Leu Ile Val Ile Lys Pro Arg Ile Ser Ser Tyr Tyr Tyr Cys Leu
        275                 280                 285
Asn Leu Thr Asn Glu Ile Leu Lys Asp Val Arg Val Arg Arg Ala Leu
    290                 295                 300
Ser Leu Ala Ile Asp Arg Asn Tyr Ile Val Glu Gln Ile Thr Arg Gly
305                 310                 315                 320
Gly Glu Lys Pro Ala Gly Ala Leu Val Pro Phe Gly Ile Ser Asp Tyr
                325                 330                 335
Glu Gly Asp Phe Arg Glu Asn Gly Gly Glu Tyr Ile Asp Ile Thr Lys
            340                 345                 350
Asp Gly Tyr Val Lys Asn Val Glu Glu Ala Lys Lys Leu Met Ala Glu
        355                 360                 365
Ala Gly Tyr Pro Asn Gly Glu Gly Phe Pro Val Met Glu Phe Lys Ala
    370                 375                 380
Asp Pro Gly Leu His Val Lys Ile Phe Glu Ala Val Gln Gln Met Trp
385                 390                 395                 400
Lys Glu Asn Leu Gly Ile Asp Val Thr Leu Thr Gln Glu Glu Trp Ala
                405                 410                 415
Val Phe Leu Gln Thr Arg Tyr Asp Arg Asn Ile Thr Met Ala Arg Gly
            420                 425                 430
Ala Trp Asn Gly Asp Phe Asp Asp Pro Val Asn Phe Met Ala Leu Cys
        435                 440                 445
Leu Ser Tyr Ser Pro Asn Asn Tyr Ser Val Tyr Ser Asn Arg Ala Tyr
    450                 455                 460
Asp Asp Met Ile Asn Glu Val Met Leu Ser Gly Asp Gln Lys Phe Arg
465                 470                 475                 480
Met Gln Thr Met His Lys Ala Glu Glu Met Leu Met Arg Glu Glu Ala

485                    490                    495
Ile Ile Pro Ile Tyr Tyr Tyr Thr Glu Pro Leu Leu Val Ser Pro Lys
            500                    505                    510
Leu Lys Asp Val Tyr Tyr Asp Ser Leu Gly Phe His Lys Phe His His
        515                    520                    525
Cys Tyr Leu Glu
    530

<210> 15
<211> 26
<212> DNA
<213> Artificial Sequence

<220>
<223> Forward primer H1-F76 sequence

<400> 15
ccattttctt tagatgaatc atatgg                                          26

<210> 16
<211> 24
<212> DNA
<213> Artificial Sequence

<220>
<223> Reverse primer  H1-R1008 sequence

<400> 16
attaggcata gatttgccaa aaat                                            24

<210> 17
<211> 23
<212> DNA
<213> Artificial Sequence

<220>
<223> Forward primer H1-F139 sequence

<400> 17
attggtattg atgctgcagc ttt                                             23

<210> 18

<211> 22

<212> DNA

<213> Artificial Sequence

<220>

<223> Reverse primer H1- R389 sequence

<400> 18

ttagcgcaac catcaaatac gt                                                22

<210> 19

<211> 30

<212> DNA

<213> Artificial Sequence

<220>

<223> Forward primer H2-F67 sequence

<400> 19

ataacacata gattctattg gaatttagaa                                        30

<210> 20

<211> 30

<212> DNA

<213> Artificial Sequence

<220>

<223> Reverse primer H2-R1209 sequence

<400> 20

tcttaatttg attatgatat caactcttct                                        30

<210> 21

<211> 22

<212> DNA

<213> Artificial Sequence

<220>

<223> Forward primer H2- F498 sequence

<400> 21
cgccaatgtt gaaactaata gc                                              22

<210> 22
<211> 25
<212> DNA
<213> Artificial Sequence

<220>
<223> Reverse primer H2- R1069 sequence

<400> 22
ctgattttgc tctttttct gataa                                            25

<210> 23
<211> 29
<212> DNA
<213> Artificial Sequence

<220>
<223> Forward primer H3-F52 sequence

<400> 23
tgtcgtaatt attcaaatac aaataactc                                       29

<210> 24
<211> 32
<212> DNA
<213> Artificial Sequence

<220>
<223> Reverse primer H3- R783 sequence

<400> 24
taaatactta tctactaatt tttgataagt gc                                   32

<210> 25
<211> 25
<212> DNA
<213> Artificial Sequence

<220>
<223> Forward primer H3- F266 sequence

<400> 25
tgcagtttga tgatcttatt acagc                                          25

<210> 26
<211> 25
<212> DNA
<213> Artificial Sequence

<220>
<223> Reverse primer H3- R543 sequence

<400> 26
gcctgcaata tcaaatctct ctact                                          25

<210> 27
<211> 26
<212> DNA
<213> Artificial Sequence

<220>
<223> Forwrad primer H5-F55 sequence

<400> 27
tgcagtaata caaacagtgg aaaatc                                         26

<210> 28
<211> 33
<212> DNA
<213> Artificial Sequence

<220>
<223> Reverse primer H5- R1071 sequence

<400> 28
atttatataa ttaattttta ctaaattcaa agg                                 33

<210> 29

<211> 25

<212> DNA

<213> Artificial Sequence

<220>

<223> Forward primer H5- F256 sequence

<400> 29

atgggagcaa aattgatagt aacac 25

<210> 30

<211> 24

<212> DNA

<213> Artificial Sequence

<220>

<223> Reverse primer H5- R847 sequence

<400> 30

aagcagcaac agcattataa acag 24

<210> 31

<211> 31

<212> DNA

<213> Artificial Sequence

<220>

<223> Forward primer H6- F70 sequence

<400> 31

ccaaatacta atacaagtac ttttgaaata g 31

<210> 32

<211> 28

<212> DNA

<213> Artificial Sequence

<220>

<223> Reverse primer H6- R783 sequence

<400> 32

ttttatataa ttaacctttg ctgaattt                                    28

<210> 33
<211> 22
<212> DNA
<213> Artificial Sequence

<220>
<223> Forward primer H6- F212 sequence

<400> 33
ttgctcaccc tgctgtagtt ag                                          22

<210> 34
<211> 25
<212> DNA
<213> Artificial Sequence

<220>
<223> Reverse primer H6-R554 sequence

<400> 34
gcaacagcat tataaacaga ttctc                                       25

<210> 35
<211> 26
<212> DNA
<213> Artificial Sequence

<220>
<223> Forward primer H11- F101 sequence

<400> 35
gcttaggttt ttcaggacat tctcat                                      26

<210> 36
<211> 26
<212> DNA
<213> Artificial Sequence

<220>

<223> Reverse primer H11-R765 sequence

<400> 36
tttctttaat tcatcaactg tttcaa                                          26

<210> 37
<211> 25
<212> DNA
<213> Artificial Sequence

<220>
<223> Forward primer H11- F265 sequence

<400> 37
aaagatactg ataaagaaat ggggc                                           25

<210> 38
<211> 30
<212> DNA
<213> Artificial Sequence

<220>
<223> Reverse primer H11-R550 sequence

<400> 38
ccttttttat ataccaatct tcttcttcag                                       30

<210> 39
<211> 22
<212> DNA
<213> Artificial Sequence

<220>
<223> Forward primer H13- F286 sequence

<400> 39
aaatggacag acggaaaaac ag                                              22

<210> 40
<211> 22
<212> DNA

<213> Artificial Sequence

<220>
<223> Reverse primer H13-R1209 sequence

<400> 40
attttctttc cacatctgct ga                                                22

<210> 41
<211> 23
<212> DNA
<213> Artificial Sequence

<220>
<223> Forward primer H13- F518 sequence

<400> 41
ctttccctac attttatccg ctt                                               23

<210> 42
<211> 20
<212> DNA
<213> Artificial Sequence

<220>
<223> Reverse primer H13-R962 sequence

<400> 42
ccgcctcttg taatctgctc                                                   20

<210> 43
<211> 35
<212> DNA
<213> Artificial Sequence

<220>
<223> NAV-H1 H1-F76-XhoI primer sequence

<400> 43
gttctcgagc cattttcttt agatgaatca tatgg                                  35

<210> 44
<211> 33
<212> DNA
<213> Artificial Sequence

<220>
<223> NAV-H1 H1-R1008-EcoRI primer sequence

<400> 44
gttgaattca ttaggcatag atttgccaaa aat                                    33

<210> 45
<211> 39
<212> DNA
<213> Artificial Sequence

<220>
<223> NAV-H2 H2-F57-XhoI primer sequence

<400> 45
ctactcgaga taacacatag attctattgg aatttagaa                              39

<210> 46
<211> 39
<212> DNA
<213> Artificial Sequence

<220>
<223> NAV-H2 H2-R1209-EcoRI primer sequence

<400> 46
gttgaattct cttaatttga ttatgatatc aactcttct                              39

<210> 47
<211> 38
<212> DNA
<213> Artificial Sequence

<220>
<223> NAV-H3 H3-F52-XhoI primer sequence

<400> 47
ctactcgagt gtcgtaatta ttcaaataca aataactc                                38

<210> 48
<211> 41
<212> DNA
<213> Artificial Sequence

<220>
<223> NAV-H3 H3-R783-EcoRI primer sequence

<400> 48
gttgaattct aaatacttat ctactaattt ttgataagtg c                            41

<210> 49
<211> 35
<212> DNA
<213> Artificial Sequence

<220>
<223> NAV-H5 H5-F55-XhoI primer sequence

<400> 49
ctactcgagt gcagtaatac aaacagtgga aaatc                                   35

<210> 50
<211> 42
<212> DNA
<213> Artificial Sequence

<220>
<223> NAV-H5 H5-R1071-EcoRI primer sequence

<400> 50
gttgaattca tttatataat taatttttac taaattcaaa gg                           42

<210> 51
<211> 40
<212> DNA
<213> Artificial Sequence

<220>

<223> NAV-H6 H6-F70-XhoI  primer sequence

<400> 51
agcctcgagc caaatactaa tacaagtact tttgaaatag                              40

<210> 52
<211> 37
<212> DNA
<213> Artificial Sequence

<220>
<223> NAV-H6 H6-R783-EcoRI primer sequence

<400> 52
gttgaattct tttatataat taacctttgc tgaattt                                 37

<210> 53
<211> 38
<212> DNA
<213> Artificial Sequence

<220>
<223> NAV-H11 H11-F4-XhoI primer sequence

<400> 53
ctactcgaga aatatatatc taatttgcat acacatac                                38

<210> 54
<211> 38
<212> DNA
<213> Artificial Sequence

<220>
<223> NAV-H11 H11-R843-EcoRI primer sequence

<400> 54
gttgaattct acacttagtc ctaatttatt tatatcaa                                38

<210> 55
<211> 35

<212> DNA
<213> Artificial Sequence

<220>
<223> NAV-H13 H13-F52-XhoI primer sequence

<400> 55
atactcgagt gttctaataa taaatcagaa gatgg                                35

<210> 56
<211> 36
<212> DNA
<213> Artificial Sequence

<220>
<223> NAV-H13 H13-R1596-EcoRI  primer sequence

<400> 56
gttgaattct tctaaataac aatgatggaa tttatg                               36

<210> 57
<211> 23
<212> DNA
<213> Artificial Sequence

<220>
<223> pTrcHis forward primer sequence

<400> 57
caatttatca gacaatctgt gtg                                             23

<210> 58
<211> 23
<212> DNA
<213> Artificial Sequence

<220>
<223> pTrcHis reverse primer sequence

<400> 58
tgcctggcag ttccctactc tcg                                             23